(19) Europäisches Patentamt
European Patent Office
Office européen des brevets

(11) EP 0 943 615 A1

(12) EUROPEAN PATENT APPLICATION
published in accordance with Art. 158(3) EPC

(43) Date of publication:
**22.09.1999 Bulletin 1999/38**

(21) Application number: **97913436.8**

(22) Date of filing: **25.11.1997**

(51) Int. Cl.[6]: **C07D 401/12**, C07D 401/14, C07D 211/90, C07D 471/04, A61K 31/445, A61K 31/495, A61K 31/535

(86) International application number:
**PCT/JP97/04287**

(87) International publication number:
**WO 98/23607 (04.06.1998 Gazette 1998/22)**

(84) Designated Contracting States:
**CH DE FR GB IT LI SE**

(30) Priority: **25.11.1996 JP 32801096**
**11.11.1997 JP 32397297**

(71) Applicant:
**Nikken Chemicals Company, Limited**
**Tokyo 104 (JP)**

(72) Inventors:
- **TASAKA, Shigeyuki,**
**Omiya Research Laboratory**
**Omiya-shi, Saitama 330 (JP)**
- **TANABE, Hirokazu,**
**Omiya Research Laboratory**
**Omiya-hi, Saitama 330 (JP)**
- **OMORI, Hiromasa,**
**Omiya Research Laboratory**
**Omiya-shi, Saitama 330 (JP)**
- **KIUE, Akira,**
**Omiya Research Laboratory**
**Omiya-shi, Saitama 330 (JP)**

(74) Representative: **Kador & Partner**
**Corneliusstrasse 15**
**80469 München (DE)**

# (54) 1,4-DIHYDROPYRIDINE DERIVATIVES

(57) A 1,4-dihydropyridine derivative having the formula (I):

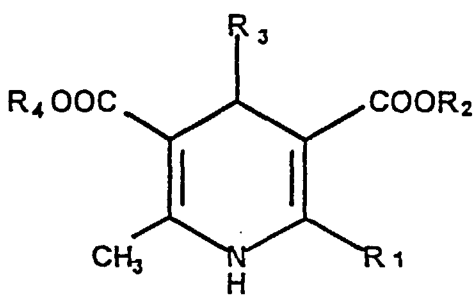

(I)

wherein, $R_1$ represents a substituted or unsubstituted phenyl or heterocyclic group, $R_2$ represents a $C_1$ to $C_5$ lower alkyl group, $R_3$ represents a substituted or unsubstituted $C_2$ to $C_8$ alkyl, alkenyl, alkynyl or substituted or unsubstituted cycloalkyl group, $R_4$ represents -A-$R_5$, wherein A represents a $C_2$ to $C_8$ alkylene group or a substituted or unsubstituted $C_2$ to $C_8$ alkenylene group, and $R_5$ represents a substituted or unsubstituted pyridyl, pyridylcarbonyl or piperadinyl group and a drug for overcoming resistance to an anti-cancer drug or a drug increasing the effect of an anti-cancer drug containing as an effective ingredient the derivative or its pharmacologically acceptable salt or hydrate.

EP 0 943 615 A1

## Description

### TECHNICAL FIELD

The present invention relates to a novel 1,4-dihydropyridine derivative having an action for overcoming resistance to an anti-cancer drug or an action for increasing the effect of an anti-cancer drug or a drug for overcoming resistance to an anti-cancer drug or a drug for increasing the effect of an anti-cancer drug having the derivative or its pharmacologically acceptable salt or hydrate as an effective ingredient.

### BACKGROUND ART

Many compounds are already known as 1,4-dihydropyridine derivatives. The majority of these known 1,4-dihydropyridine derivatives have a pharmacological activity with respect to the circulatory system. For other pharmacological activities, there have been just a few reports for those having an anti-inflammatory action, liver protecting action, etc.

On the other hand, "acquired resistance", where the effect of the anti-cancer drug is lost during the treatment, is becoming a problem in the chemotherapy of cancers. Multidrug resistance which exhibited against a variety of anti-cancer drugs is becoming an important problem. As a method for overcoming this multidrug resistance, it has been reported that combined administration of an anti-cancer drug and some calcium antagonists (1,4-dihydropyridine compounds such as nicardipine) is effective (*Cancer Res.*, 41, 1967-1972 (1981), *Cancer and Chemotherapy*, vol. 11, 750-759 (1984)).

Further, Japanese Unexamined Patent Publication (Kokai) No. 2-40383, Japanese Unexamined Patent Publication (Kokai) No. 2-240081, Japanese Examined Patent Publication (Kokoku) No. 6-92391, Japanese Examined Patent Publication (Kokoku) No. 6-92401; etc. describe a 1,4-dihydropyridine compound having a dioxene ring or a dithiene ring at its 4-position, Japanese Unexamined Patent Publication (Kokai) No. 5-117235 and Japanese Unexamined Patent Publication (Kokai) No. 2-138221 describes a 1,4-dihydropyridine compound having an aromatic group such as a phenyl group bonded at its 4-position, and WO96/04268 describes a 1,4-dihydropyridine compound having, an alkyl group etc., bonded at its 4-position, as those having an action for overcoming resistance to an anti-cancer drug.

However, the methods described in the above references (i.e., *Cancer Res.*, 41, 1967-1972 (1981) and *Cancer and Chemotherapy*, vol. 11, 750 to 759 (1984)) use calcium antagonists for overcoming resistance to anti-cancer drugs and have the defect that they are not necessarily practical in terms of side effects. That is, calcium channel blockers inherently have a powerful action and act on the heart, blood vessels, etc. even in very small amounts, and therefore, if large amounts of these drugs are used, there is the problem that inconvenient effects might be caused on the heart and circulatory system.

Further, among the 1,4-dihydropyridines described in the above references, Japanese Unexamined Patent Publication (Kokai) No. 2-40383, Japanese Unexamined Patent Publication (Kokai) No. 2-240081, WO96/04268, etc., there are preferable compounds having an action to increase the effect of anti-cancer drugs or an action for overcoming the resistance to an anti-cancer drug and further have almost no calcium channel blocking action. However, the chemical structures of the 1,4-dihydropyridine compounds described in these publications are clearly different from those of the present invention. There has been no description at all of a compound substituted with an alkyl group etc. at the 4-position and substituted a heterocyclic group such as a pyridyl group at the 2-position.

### DISCLOSURE OF THE INVENTION

Therefore, an object of the present invention is to develop a 1,4-dihydropyridine derivative capable of overcoming the problems of a conventional drug for overcoming resistance to an anti-cancer drug or a drug for increasing the effect of an anti-cancer drug and suitable for these applications.

In accordance with the present invention, there is provided a 1,4-dihydropyridine derivative having the formula (I):

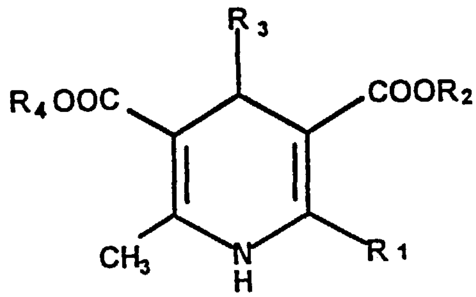

(I)

wherein $R_1$ represents a substituted or unsubstituted phenyl or heterocyclic group, $R_2$ represents a $C_1$ to $C_5$ lower alkyl group, $R_3$ represents a substituted or unsubstituted $C_2$ to $C_8$ alkyl, alkenyl or alkynyl group, or substituted or unsubstituted cycloalkyl group, $R_4$ represents -A-$R_5$, wherein A represents a $C_2$ to $C_8$ alkylene group or substituted or unsubstituted $C_2$ to $C_8$ alkenylene group, and $R_5$ represents a substituted or unsubstituted pyridyl, pyridylcarbonyl or piperadinyl group and also a drug, particularly a drug for overcoming resistance to an anti-cancer drug or a drug for increasing the effect of an anti-cancer drug containing, as an effective ingredient, the derivative or its pharmacologically acceptable salt or hydrate.

BEST MODE FOR CARRYING OUT THE INVENTION

The present inventors synthesized 1,4-dihydropyridine derivatives having various substituents and screened a broad range of these compounds for combined effects with anti-cancer drugs. As a result, we found a novel compound, that is, a 1,4-dihydropyridine derivative having the formula (I) has the action for remarkably increasing the sensitivity of cancer cells to an anti-cancer drug, in particular, the sensitivity of acquiring resistant cancer cells to an anti-cancer drug (i.e., the action for overcoming resistance to anti-cancer drug). Further, we found that such compounds exhibit an effect for prolonging the survival days of a cancerous animal by the combined administration of an anti-cancer drug and further that there are almost no action for the calcium channel, and the toxicities are low, and thus the present invention has been completed.

The present invention will now be explained in further detail.

In the 1,4-dihydropyridine derivative having the formula (I), as $R_1$, a substituted or unsubstituted phenyl or heterocyclic group may be mentioned. Preferably, phenyl, pyridyl, or a phenyl group substituted with one heterocyclic group selected from imidazopyridine, piperadine, imidazole, morpholine, indole, benzimidazole, and 1H-benzotriazole, more specifically, phenyl, 3-pyridyl, 4-(-2-methylimidazo[4,5-b]pyridin-1-yl)phenyl, 4-(2-methylimidazo[4,5-c]pyridin-1-yl)phenyl, 4-(4-methylpiperadin-1-yl)phenyl, 4-methylthiophenyl, 4-(1H-benzotriazol-1-yl)phenyl, 4-(benzimidazoyl-1-yl)phenyl, 4-(1-imidazolyl)phenyl, 3-(1-imidazolyl)phenyl, 4-morpholinophenyl, 4-(1-indolyl)phenyl, etc. may be mentioned.

In the formula (I), as $R_2$, a $C_1$ to $C_5$ lower alkyl group, preferably, methyl, ethyl, or propyl, may be mentioned.

In the formula (I), as $R_3$, a substituted or unsubstituted $C_2$ to $C_8$ alkyl, alkenyl or alkynyl group or a substituted or unsubstituted cycloalkyl group, preferably a $C_3$ to $C_6$ alkyl or alkenyl group; a $C_1$ to $C_3$ lower alkyl group, $C_2$ to $C_3$ lower alkenyl group, or $C_2$ to $C_3$ lower alkynyl group substituted with a phenyl group, thienyl group, furyl group, cyclohexyl group, cyclohexylidene group, etc.; or cycloalkyl group may be mentioned. Specifically, isopropyl, n-pentyl, n-butyl, 2, 2-dimethylpropyl, 2-methylpropyl, 2-methyl-1-propenyl, benzyl, phenetyl, 3-phenylpropyl, 2-phenylethenyl, 2-phenylethynyl, 2-thienylethenyl, 2-furylethenyl, 2-cyclohexylethenyl, cyclohexyl etc. may be mentioned.

In the formula (I), $R_4$ represents -A-$R_5$, wherein A represents a $C_2$ to $C_8$ alkylene group or substituted or unsubstituted $C_2$ to $C_8$ alkenylene group, and $R_5$ represents a substituted or unsubstituted pyridyl group or piperadinyl group. Preferably, A represents a $C_3$ to $C_6$ linear alkylene or $C_3$ to $C_6$ alkenylene group substituted or unsubstituted with a heterocyclic group such as a thienyl group and $R_5$ represents a 3-pyridyl, 3-pyridylcarbonyl or piperadinyl group substituted with phenyl, benzyl, benzhydryl, or 1-phenyl-1-pyridylmethyl, etc. Particularly preferably, A represents trimethylene, pentamethylene, propenylene, or a propenylene group substituted with a heterocyclic group such as thienyl group and $R_5$ represents a 3-pyridyl, nicotinoyl, 4-benzhydrylpiperadin-1-yl, etc.

In the present invention, as specifically preferable compounds, all of the compounds shown in the Examples may be mentioned.

The 1,4-dihydropyridine derivative having the formula (I) of the present invention may be produced by a known method used for the production of a 1,4-dihydropyridine derivative in the past.

That is, the compound having the formula (I) may be produced by a reaction of an aldehyde having the formula

(II) and an acetoacetate ester having the formula (III) and 3-aminocrotonate ester having the formula (IV) in the presence or absence of an organic solvent:

$$\underset{(II)}{R_3CHO} + \underset{(III)}{R_1COCH_2COOR_2} + \underset{(IV)}{CH_3C(NH_2)=CHCOOR_4} \longrightarrow (I)$$

wherein $R_1$, $R_2$, $R_3$, $R_4$, etc. are the same as defined in the formula (I).

The reactions used in these production methods are basically the same as the known methods for the production of a 1,4-dihydropyridine derivative in the past (e.g., the methods described in Japanese Examined Patent Publication (Kokoku) No. 46-40625 and No. 56-37225, Japanese Unexamined Patent Publication (Kokai) No. 60-214786, etc.). Therefore, the 1,4-dihydropyridine derivative of the present invention may be produced by suitably applying a different reaction described in these known references in addition to the above method.

The starting materials used for these methods are all known and can be readily obtained or produced by a person skilled in the art depending upon the need. For example, an acetoacetate ester may be produced by a reaction with a diketene and an alcohol. Further, a 3-aminocrotonate ester may be produced by the reaction of ammonia gas with the above acetoacetate ester. An aldehyde can be readily produced by a known method widely used, that is, the reduction of an ester or the oxidation of an alcohol.

A compound having the formula (I) obtained by this method can be isolated and purified by a known treatment means (e.g., extraction, chromatography, recrystallization, etc.)

The 1,4-dihydropyridine derivative having the formula (I) has asymmetric carbon atoms, and therefore, have optical isomers. The present invention includes all optical isomers and mixtures thereof. Further, a compound having an alkenyl group sometimes has geometrical isomers in addition to optical isomers. The present invention includes all of these isomers and mixtures thereof isomers too. Further, the mixtures of isomers may be separated into the chiral isomers depending upon the need by a separated crystallization or chromatography.

The compound according to the present invention exhibits an action for increasing the effect of an anti-cancer drug and exhibits an action for overcoming the resistance to anti-cancer drugs for an adriamycin resistant cancer or vincristine resistant cancer and prolongs the survival days of a cancerous animal by the combined administration of an anti-cancer drugs, and therefore, is useful as a drug for overcoming resistance to an anti-cancer drug or drug for increasing the effect of an anti-cancer drug.

When the compound according to the present invention is used as a drug for overcoming resistance to an anti-cancer drug or a drug for increasing the effect of an anti-cancer drug, it may be administered by a suitable method such as oral or nonoral administration. As a form of oral administration are tablets, granules, capsules, pills, dispersions, liquids, etc. Further as a form of non-oral administration are injections, suppositories, etc. These may be prepared according to ordinary methods using the present compound or its pharmacologically acceptable salt with a usual preparation carrier.

In the case of oral administration, the preparations can be prepared into the desired form using an excipient such as lactose, glucose, corn starch, sucrose, a disintegrator such as calcium carboxymethylcellulose, hydroxypropylcellulose, a lubricant such as calcium stearate, magnesium stearate, talc, polyethylene glycol, hardened oil, a binder such as hydroxypropylcellulose, hydroxypropylmethylcellulose, carboxymethylcellulose, polyvinyl alcohol, gelatin, arabia gum, a humectant such as glycerin, ethylene glycol, and, in addition, if necessary, a surfactant, a corrigent, etc.

In the case of a non-oral drug, a diluent such as water, ethanol, glycerol, propylene glycol, polyethylene glycol, agar, tragacanth gum may be used and, depending upon the need, a solution adjuvant, a buffer agent, a preservative, a flavor, a coloring agent, etc. may be used.

When formulating the present compound as a drug for overcoming resistance to an anti-cancer drug or a drug for increasing the effect of an anti-cancer drug, the dosage, as the present compound, is per adult, in the case of oral administration, 5 to 1000 mg per day, preferably 5 to 200 mg, and in the case of non-oral administration, 1 to 500 mg per day, preferably 1 to 200 mg. The desired effect of treatment can be expected by administration divided into one to three dosages per day.

## Examples

The following Synthesis Examples, Preparation Examples, and Test Examples of the compounds according to

the present invention will now be explained as Examples, but the present invention is of course not limited to these Examples:

Synthesis Examples

Synthesis Examples are shown below. The NMR data shows the signals of $^1$H-NMR measured by a $CDCl_3$ solvent.

Example 1

Synthesis of 4-isopropyl-6-methyl-2-[4-(2-methylimidazo[4,5-b]pyridin-1-yl)phenyl]-1,4-dihydropyridin-3,5-dicarboxylate 3-ethylester, 5-[3-(3-pyridyl)propyl]ester (Compound 1)

1.5 g of ethyl 4'-(2-methylimidazo[4,5-b]pyridin-1-yl)benzoylacetate, 1.1 g of 3-aminocrotonate 3-(3-pyridyl)propylester, and 0.4 g of isobutyl aldehyde were heated and refluxed in 20 ml of isopropanol for 6 hours. After the reaction, the reaction solution was condensed to dryness in vacuo and extracted with ethyl acetate. The extracted solution was washed with water, then dried over anhydrous magnesium sulfate and evaporated in vacuo. The oily substance was purified by silica gel column chromatography to obtain the target compound in an amount of 0.67 g (23%).

NMR: 0.92 (6H, m), 1.03 (3H, t), 1.80 (1H, m), 2.05 (2H, m), 2.38 (3H, S), 2.63 (3H, s), 2.78 (2H, t), 4.01 (2H, m), 4.12 (1H, d), 4.20 (2H, m), 7.25 (2H, m), 7.49 (2H, m), 7.56 (3H, m), 8.03 (1H, m), 8.31 (1H, m), 8.46 (1H, m), 8.50 (1H, d)

Example 2

Synthesis of 4-isopronyl-6-methyl-2-[4-(2-methylimidazo[4,5-c]pyridin-1-yl)phenyl]-1,4-dihydronyridin-3,5-dicarboxylate 3-ethylester, 5-[3-(3-pyridyl)propyl]ester (Compound 2)

1.0 g of ethyl 4'-(2-methylimidazo(4,5-c]pyridin-1-yl)benzoylacetate, 0.7 g of 3-aminocrotonate 3-(3-pyridyl)propylester, and 0.3 g of isobutyl aldehyde were heated and refluxed in 20 ml of ethanol for 5 hours. The mixture was cooled to room temperature, added with 1.1 g of ammonium chloride, then 0.4 g of potassium hydroxide, then further heated and refluxed for 2 hours. After the reaction, the reaction solution was concentrated to dryness in vacuo and the oily substance was purified by silica gel column chromatography to obtain the target compound in an amount of 1.1 g (62%).

NMR: 0.93 (6H, d), 1.05 (3H, t), 1.81 (1H, m), 2.05 (2H, m), 2.42 (3H, s), 2.59 (3H, s), 2.79 (2H, t), 4.02 (2H, m), 4.13 (1H, d), 4.21 (2H, m), 7.11 (1H, d), 7.24 (1H, m), 7.42 (2H, d), 7.55 (1H, d), 7.59 (2H, d), 8.37 (1H, d), 8.47 (1H, m), 8.50 (1H, m), 9.06 (1H, s)

The compounds of the following Examples synthesized on Example 1 or Example 2 will now be given together with the materials used and the NMR data. Further, the compounds were purified by recrystallizing with a suitable solvent or by silica gel column chromatography if necessary.

Example 3

Synthesis of 6-methyl-2-[4-(2-methylimidazo[4,5-b]pyridin-1-yl)phenyl]-4-pentyl-1,4-dihydropyridin-3,5-dicarboxylate 3-ethylester, 5-[3-(3-pyridyl)propyl]ester (Compound 3)

Starting material

Ethyl 4'-(2-methylimidazo[4,5-b]pyridin-1-yl)benzoylacetate
3-Aminocrotonate 3-(3-pyridyl)propylester
Capronaldehyde

NMR: 0.87 (3H, t), 1.03 (3H, t), 1.30 (6H, m), 1.51 (2H, m), 2.05 (2H, m), 2.36 (3H, s), 2.62 (3H, s), 2.78 (2H, t), 4.01 (2H, m), 4.13 (1H, t), 4.20 (2H, m), 7.23 (2H, m), 7.49 (2H, d), 7.56 (3H, m), 8.03 (1H, m), 8.31 (1H, m), 8.46 (1H, m), 8.49 (1H, d)

Example 4

Synthesis of 6-methyl-2-[4-(2-methylimidazo[4,5-b]pyridin-1-yl)phenyl]-4-pentyl-1,4-dihydropyridin-3,5-dicarboxylate 3-ethylester, 5-[3-(3-pyridyl)-2-propenyl]ester (Compound 4)

Starting material

Ethyl 4'-(2-methylimidazo[4,5-b]pyridin-1-yl)benzoylacetate
3-Aminocrotonate 3-(3-pyridyl)-2-propenylester
Capronaldehyde

NMR: 0.81 (3H, t), 1.17 (3H, t), 1.33 (8H, m), 2.51 (3H, s), 2.61 (3H, s), 3.50 (1H, t), 4.10 (1H, t), 4.15 (2H, m), 4.91 (2H, m), 6.44 (1H, m), 6.71 (1H, d), 7.27 (2H, m), 7.56 (2H, d), 7.74 (1H, m), 8.03 (1H, m), 8.21 (2H, d), 8.33 (1H, m), 8.51 (1H, m), 8.64 (1H, d)

Example 5

Synthesis of 6-methyl-2-[4-(2-methylimidazo[4,5-b]pyridin-1-yl)phenyl]-4-pentyl-1,4-dihydropyridin-3,5-dicarboxylate 3-ethylester, 5-[3-(4-benzhydrylpiperadin-1-yl)propyl]ester (Compound 5)

Starting material

Ethyl 4'-(2-methylimidazo[4,5-b]pyridin-1-yl)benzoylacetate
3-Aminocrotonate 3-(4-benzhydrylpiperadin-1-yl)propylester
Capronaldehyde

NMR: 0.87 (3H, t), 1.26 (3H, t), 1.32 (8H, m), 1.47 (2H, m), 1.88 (2H, m), 2.32 (3H, s), 2.46 (8H, m), 2.62 (3H, s), 4.00 (2H, m), 4.07 (1H, t), 4.19 (2H, m), 4.22 (1H, s), 7.17 (2H, m), 7.26 (2H, m), 7.42 (4H, m), 7.48 (2H, d), 7.54 (2H, d), 8.03 (1H, m), 8.31 (1H, m)

Example 6

Synthesis of 4-butyl-6-methyl-2-[4-(2-methylimidazo[4,5-c]pyridin-1-yl)phenyl]-1,4-dihydropyridin-3,5-dicarboxylate 3-ethylester, 5-[3-(3-pyridyl)propyl]ester (Compound 6)

Starting material

Ethyl 4'-(2-methylimidazo[4,5-c]pyridin-1-yl)benzoylacetate
3-Aminocrotonate 3-(3-pyridyl)propylester
Valeryl aldehyde

NMR: 0.91 (3H, t), 1.04 (3H, t), 1.30 - 1.67 (8H, m), 2.05 (2H, m), 2.40 (3H, s), 2.58 (3H, s), 2.78 (2H, t), 4.02 (2H, m), 4.13 (1H, t), 4.21 (2H, m), 7.09 (1H, d), 7.23 (1H, m), 7.40 (2H, d), 7.54 (1H, m), 7.57 (2H, d), 8.34 (1H, d), 8.46 (1H, m), 8.48 (1H, m), 9.05 (1H, s)

Example 7

Synthesis of 6-methyl-2-[4-(2-methylimidazo[4,5-c]pyridin-1-yl)phenyl]-4-pentyl-1,4-dihydropyridin-3,5-dicarboxylate 3-ethylester, -5-[3-(3-pyridyl)propyl]ester (Compound 7)

Starting material

Ethyl 4'-(2-methylimidazo[4,5-c]pyridin-1-yl)benzoylacetate
3-Aminocrotonate 3-(3-pyridyl)propylester
Capronaldehyde

NMR: 0.88 (3H, t), 1.05 (3H, t), 1.30 - 1.62 (8H, m), 2.05 (2H, m), 2.39 (3H, s), 2.59 (3H, s), 2.78 (2H, t), 4.02 (2H, m), 4.14 (1H, t), 4.20 (2H, m), 7.10 (1H, d), 7.23 (1H, m), 7.41 (2H, d), 7.54 (1H, m), 7.57 (2H, d), 8.36

(1H, d), 8.46 (1H, m), 8.48 (1H, m), 9.06 (1H, s)

Example 8

Synthesis of 4-(2, 2-dimethylpropyl)-6-methyl-2-[4-(2-methylimidazo[4,5-c]pyridin-1-yl)phenyl]-1,4-dihydropyridin-3,5-dicarboxylate 3-ethylester, 5-[3-(3-pyridyl)propyl]ester (Compound 8)

Starting material

Ethyl 4'-(2-methylimidazo[4,5-c]pyridin-1-yl)benzoylacetate
3-Aminocrotonate 3-(3-pyridyl)propylester
3,3-Dimethylbutylaldehyde

NMR: 0.88 (3H, t), 1.02 (9H, s), 1.07 (3H, t), 1.33 (1H, m), 1.49 (1H, m), 2.06 (2H, m), 2.44 (3H, s), 2.58 (3H, s), 2.81 (2H, t), 4.03 (2H, m), 4.22 (2H, m), 4.33 (1H, t), 7.09 (1H, d), 7.24 (1H, m), 7.39 (2H, d), 7.55 (1H, m), 7.59 (2H, d), 8.31 (1H, d), 8.46 (1H, d), 8.50 (1H, s), 9.04 (1H, s)

Example 9

Synthesis of 4-cyclohexyl-6-methyl-2-[4-(2-methylimidazo[4,5-c]pyridin-1-yl)phenyl]-1,4-dihydropyridin-3,5-dicarboxylate 3-ethylester, 5-[3-(3-pyridyl)propyl]ester (Compound 9)

Starting material

Ethyl 4'-(2-methylimidazo[4,5-c]pyridin-1-yl)benzoylacetate
3-Aminocrotonate 3-(3-pyridyl)propylester
Cyclohexanal

NMR: 1.04 (3H, t), 1.06 - 2.02 (11H, m), 2.05 (2H, m), 2.44 (3H, s), 2.57 (3H, s), 2.80 (2H, t), 4.01 (2H, m), 4.13 (1H, d), 4.21 (2H, m), 7.06 (1H, d), 7.24 (1H, m), 7.37 (2H, d), 7.55 (1H, m), 7.59 (2H, d), 8.25 (1H, m), 8.45 (1H, d), 8.50 (1H, s), 9.02 (1H, s)

Example 10

Synthesis of 6-methyl-2-[4-(2-methylimidazo[4,5-c]pyridin-1-yl)phenyl]-4-(2-methylpropyl)-1,4-dihdropyridin-3,5-dicarboxylate 3-ethylester, 5-[3-(3-pyridyl)propyl]ester (Compound 10)

Starting material

Ethyl 4'-(2-methylimidazo[4,5-c]pyridin-1-yl)benzoylacetate
3-Aminocrotonate 3-(3-pyridyl)propylester
Isovaleryl aldehyde

NMR: 0.97 (6H, d), 1.00 (2H, d), 1.07 (3H, t), 1.33 (1H, m), 1.65 (1H, m), 2.06 (2H, m), 2.40 (3H, s), 2.59 (3H, s), 2.79 (2H, t), 4.03 (2H, m), 4.16 (1H, t), 4.20 (2H, m), 7.11 (1H, d), 7.24 (1H, m), 7.41 (2H, d), 7.54 (1H, m), 7.58 (2H, d), 8.37 (1H, d), 8.46 (1H, m), 8.47 (1H, s), 9.07 (1H, s)

Example 11

Synthesis of 6-methyl-2-[4-(2-methylimidazo[4,5-c]pyridin-1-yl)phenyl]-4-(2-methyl-1-propenyl)-1,4-dihydropyridin-3,5-dicarboxylate 3-ethylester, 5-[3-(3-pyridyl)propyl]ester (Compound 11)

Starting material

Ethyl 4'-(2-methylimidazo[4,5-c]pyridin-1-yl)benzoylacetate
3-Aminocrotonate 3-(3-pyridyl)propylester
3-Methyl-2-butenal

NMR: 1.05 (3H, t), 1.70 (3H, s), 1.88 (1H, s), 2.04 (2H, m), 2.39 (3H, s), 2.58 (3H, s), 2.76 (2H, t), 4.02 (2H, q), 4.21 (2H, m), 4.80 (1H, d), 5.16 (1H, d), 7.09 (1H, d), 7.24 (1H, m), 7.39 (2H, d), 7.53 (1H, d), 7.59 (2H, d), 8.34 (1H, d), 8.46 (1H, d), 8.47 (1H, s), 9.05 (1H, s)

Example 12

Synthesis of 4-benzyl-6-methyl-2-[4-(2-methylimidazo[4,5-c]pyridin-1-yl)phenyl]-1,4-dihydropyridin-3,5-dicarboxylate 3-ethylester, 5-[3-(3-pyridyl)propyl]ester (Compound 12)

Starting material

Ethyl 4'-(2-methylimidazo[4,5-c]pyridin-1-yl)benzoylacetate
3-Aminocrotonate 3-(3-pyridyl)propylester
Phenylacetoaldehyde

NMR: 1.02 (3H, s), 2.03 (2H, m), 2.30 (3H, s), 2.55 (3H, s), 2.77 (2H, m), 2.83 (2H, m), 3.95 (2H, m), 4.14 (2H, m), 4.44 (1H, t), 7.02 (1H, d), 7.16 - 7.30 (7H, m), 7.31 (2H, d), 7.42 (2H, d), 7.56 (1H, d), 8.22 (1H, d), 8.45 (1H, d), 8.49 (1H, s), 8.98 (1H, s)

Example 13

Synthesis of 6-methyl-2-[4-(2-methylimidazo[4,5-c]pyridin-1-yl)phenyl]-4-(2-phenylethyl)-1,4-dihydropyridin-3,5-dicarboxylate 3-ethylester, 5-[3-(3-pyridyl)propyl]ester (Compound 13)

Starting material

Ethyl 4'-(2-methylimidazo[4,5-c]pyridin-1-yl)benzoylacetate
3-Aminocrotonate 3-(3-pyridyl)propylester
3-Phenylpropionaldehyde

NMR: 1.04 (3H, s), 1.90 (2H, m), 2.04 (2H, m), 2.42 (3H, s), 2.59 (3H, s), 2.76 (2H, m), 4.03 (2H, q), 4.21 (2H, m), 4.27 (1H, t), 7.09 (1H, d), 7.17 - 7.27 (7H, m), 7.40 (2H, d), 7.51 (1H, m), 7.55 (2H, d), 8.34 (1H, d), 8.45 (1H, m), 8.47 (1H, s), 9.05 (1H, s)

Example 14

Synthesis of 6-methyl-2-[4-(2-methylimidazo[4,5-c]pyridin-1-yl)phenyl]-4-[(E)-2-phenylethenyl]-1,4-dihydropyridin-3,5-dicarboxylate 3-ethylester, 5-[3-(3-pyridyl)propyl]ester (Compound 14)

Starting material

Ethyl 4'-(2-methylimidazo[4,5-c]pyridin-1-yl)benzoylacetate
3-Aminocrotonate 3-(3-pyridyl)propylester
Cinnamaldehyde

NMR: 1.04 (3H, t), 2.03 (2H, m), 2.47 (3H, s), 2.58 (3H, s), 2.75 (2H, t), 4.03 (2H, q), 4.16 (2H, m), 4.26 (1H, m), 4.84 (1H, d), 6.36 (1H, d), 6.45 (1H, d), 7.07 (1H, d), 7.13 - 7.45 (8H, m), 7.38 (2H, d), 7.60 (2H, d), 8.28 (1H, d), 8.42 (1H, s), 9.04 (1H, s)

Example 15

Synthesis of 6-methyl-4-pentyl-2-phenyl-1,4-dihydropyridin-3,5-dicarboxylate 3-ethylester, 5-[3-(3-pyridyl)propyl]ester (Compound 15)

Starting material

Ethyl benzoylacetate
3-Aminocrotonate 3-(3-pyridyl)propylester

Capronaldehyde

NMR: 0.86 (3H, t), 0.91 (3H, t), 1.28 - 1.49 (8H, m), 2.02 (2H, m), 2.33 (3H, s), 2.76 (2H, t), 3.92 (2H, m), 4.08 (1H, t), 4.17 (2H, m), 7.22 (1H, m), 7.30 (2H, m), 7.40 (3H, m), 7.52 (1H, d), 8.44 (1H, d), 8.47 (1H, s)

Example 16

Synthesis of 6-methyl-4-pentyl-2-(3-pyridyl)-1,4-dihydropyridin-3,5-dicarboxylate 3-ethylester, 5-[3-(3-pyridyl)propyl]ester (Compound 16)

Starting material

Ethyl nicotinoylacetate
3-Aminocrotonate 3-(3-pyridyl)propylester
Capronaldehyde

NMR: 0.85 (3H, t), 0.96 (3H, t), 1.24 - 1.50 (8H, m), 2.30 (2H, m), 2.35 (3H, s), 2.76 (2H, t), 3.94 (2H, m), 4.11 (1H, t), 4.19 (2H, m), 7.23 (1H, m), 7.33 (1H, m), 7.53 (1H, m), 7.64 (1H, m), 8.43 - 8.47 (2H, m), 8.53 (1H, s), 8.61 (1H, m)

Example 17

Synthesis of 6-methyl-2-[4-(2-methylimidazo[4,5-c]pyridin-1-yl)phenyl]-4-pentyl-1,4-dihydropyridin-3,5-dicarboxylate 3-ethylester, 5-[5-(3-pyridyl)pentyl]ester (Compound 17)

Starting material

Ethyl 4'-(2-methylimidazo[4,5-c] pyridin-1-yl)benzoylacetate
3-Aminocrotonate 5-(3-pyridyl)pentylester
Capronaldehyde

NMR: 0.89 (3H, t), 1.04 (3H, t), 1.30 - 1.51 (8H, m), 1.73 (4H, m), 2.38 (3H, s), 2.57 (3H, s), 2.65 (2H, t), 4.01 (2H, m), 4.10 (1H, t), 4.17 (2H, m), 7.07 (1H, d), 7.22 (1H, m), 7.37 (2H, d), 7.50 (1H, d), 7.56 (2H, d), 8.29 (1H, m), 8.44 (1H, d), 8.45 (1H, s), 9.03 (1H, s)

Example 18

Synthesis of 6-methyl-2-[4-(2-methylimidazo[4,5-c]pyridin-1-yl)phenyl]-4-(2-phenylethyl)-1,4-dihydropyridin-3,5-dicarboxylate 3-ethylester, 5-[5-(3-pyridyl)pentyl]ester (Compound 18)

Starting material

Ethyl 4'-(2-methylimidazo[4,5-c]pyridin-1-yl)benzoylacetate
3-Aminocrotonate 5-(3-pyridyl)pentylester
3-Phenylpropionaldehyde

NMR: 1.03 (3H, t), 1.48 (2H, m), 1.73 (4H, m), 1.86 (2H, m), 2.40 (3H, s), 2.58 (3H, s), 2.62 (2H, t), 2.72 (2H, m), 4.02 (2H, q), 4.21 (2H, m), 4.23 (1H, t), 7.08 (1H, d), 7.17 - 7.50 (7H, m), 7.20 (2H, d), 7.54 (2H, d), 8.30 (1H, m), 8.42 (1H, d), 8.43 (1H, s), 9.04 (1H, s)

Example 19

Synthesis of 6-methyl-2-[4-(2-methylimidazo[4,5-c]pyridin-1-yl)phenyl]-4-pentyl-1,4-dihydropyridin-3,5-dicarboxylate 3-ethylester, 5-[(Z)-3-(3-pyridyl)-3-(2-thienyl)-2-propenyl]ester (Compound 19)

Starting material

Ethyl 4'-(2-methylimidazo[4,5-c]pyridin-1-yl)benzoylacetate

3-Aminocrotonate[(Z)-3-(3-pyridyl)-3-(2-thienyl)-2-propenyl]ester
Capronaldehyde

NMR: 0.87 (3H, t), 1.05 (3H, t), 1.30 - 1.53 (8H, m), 2.38 (3H, s), 2.58 (3H, s), 4.02 (2H, q), 4.11 (1H, t), 4.64 (2H, m), 6.43 (1H, t), 6.68 (1H, d), 6.95 (1H, t), 7.09 (1H, d), 7.25 (1H, s), 7.37 (1H, m), 7.41 (2H, d), 7.56 (2H, d), 7.69 (1H, m), 8.34 (1H, d), 8.59 (1H, s), 8.65 (1H, d), 9.06 (1H, s)

Example 20

Synthesis of 6-methyl-2-[4-(2-methylimidazo[4,5-c]pyridin-1-yl)phenyl]-4-(2-phenylethyl)-1,4-dihydropyridin-3,5-dicarboxylate 3-ethylester, 5-[(Z]-3-(3-pyridyl)-3-(2-thienyl)-2-propenyl]ester (Compound 20)

Starting material

Ethyl 4'-(2-methylimidazo[4,5-c]pyridin-1-yl)benzoylacetate
3-Aminocrotonate [(Z)-3-(3-pyridyl)-3-(2-thienyl)-2-propenyl]ester
3-Phenylpropionaldehyde

NMR: 1.04 (3H, t), 1.89 (2H, m), 2.40 (3H, s), 2.58 (3H, s), 2.73 (2H, m), 4.03 (2H, q), 4.24 (1H, t), 4.64 (2H, m), 6.41 (1H, t), 6.67 (1H, d), 6.95 (1H, t), 7.09 (1H, d), 7.15 - 7.26 (5H, m), 7.35 (1H, m), 7.39 (2H, d), 7.54 (2H, d), 7.67 (1H, m), 8.33 (1H, d), 8.59 (1H, s), 8.64 (1H, d), 9.05 (1H, s)

Example 21

Synthesis of 6-methyl-2-[4-(2-methylimidazo[4,5-c]pyridin-1-yl)phenyl]-4-[(E)-2-phenylethenyl]-1,4-dihydropyridin-3,5-dicarboxylate 3-ethylester, 5-[5-(3-pyridyl)pentyl]ester (Compound 21)

Starting material

Ethyl 4'-(2-methylimidazo[4,5-c]pyridin-1-yl)benzoylacetate
3-Aminocrotonate 5-(3-pyridyl)pentylester
Cinnamaldehyde

NMR: 1.03 (3H, t), 1.45 (2H, m), 1.63 (2H, m), 1.73 (2H, m), 2.42 (3H, s), 2.53 (2H, t), 2.59 (3H, s), 4.02 (2H, m), 4.13 (1H, m), 4.23 (1H, t), 4.80 (1H, d), 6.30 (1H, dd), 6.41 (1H, d), 7.10 (1H, d), 7.17 - 7.50 (7H, m), 7.29 (2H, t), 7.41 (2H, d), 7.60 (2H, d), 8.30 (1H, m), 8.42 (1H, d), 8.43 (1H, s), 9.04 (1H, s)

Example 22

Synthesis of 4-[(E)-2-(2-chlorophenyl]ethenyl]-6-methyl-2-[4-(2-methylimidazo[4,5-b]pyridin-1-yl)phenyl]-1,4-dihydropyridin-3,5-dicarboxylate 3-ethylester, 5-[(E)-3-(3-pyridyl)-2-propenyl]ester (Compound 22)

Starting material

Ethyl 4'-(2-methylimidazo[4,5-b]pyridin-1-yl)benzoylacetate
3-Aminocrotonate (E)-3-(3-pyridyl)-2-propenylester
2'-Chlorocinnamaldehyde

NMR: 1.03 (3H, t), 2.42 (3H, s), 2.63 (3H, s), 4.02 (2H, q), 4.88 (2H, m), 6.30 (1H, d), 6.34 (1H, d), 6.46 (1H, dt), 6.67 (1H, d), 6.86 (1H, d), 7.13 - 7.32 (5H, m), 7.51 (2H, d), 7.55 (1H, d), 7.62 (2H, d), 7.67 (1H, d), 8.03 (1H, d), 8.31 (1H, d), 8.47 (1H, dd), 8.56 (1H, d)

Example 23

Synthesis of 6-methyl-2-[4-(2-methylimidazo[4,5-c]pyridin-1-yl)phenyl]-4-pentyl-1,4-dihydropyridin-3,5-dicarboxylate 3-ethylester, 5-[(E)-3-(3-pyridyl)-2-propenyl]ester (Compound 23)

Starting material

Ethyl 4'-(2-methylimidazo[4,5-c]pyridin-1-yl)benzoylacetate
3-Aminocrotonate (E)-3-(3-pyridyl)-2-propenylester
Capronaldehyde

NMR: 0.86 (3H, t), 1.03 (3H, t), 1.28 - 1.55 (8H, m), 2.42 (3H, s), 2.58 (3H, s), 4.01 (2H, m), 4.16 (1H, t), 4.87 (2H, m), 6.45 (1H, dt), 6.70 (1H, d), 7.08 (1H, d), 7.28 (1H, m), 7.38 (2H, d), 7.56 (2H, d), 7.74 (1H, m), 8.31 (1H, d), 8.50 (1H, dd), 8.63 (1H, d), 9.04 (1H, s)

Example 24

Synthesis of 6-methyl-2-[4-(2-methylimidazo[4,5-c]pyridin-1-yl)phenyl]-4-(2-phenyl)ethyl-1,4-dihydropyridin-3,5-dicarboxylate 3-ethylester, 5-[(E)-3-(3-pyridyl)-2-propenyl]ester (Compound 24)

Starting material

Ethyl 4'-(2-methylimidazo[4,5-c]pyridin-1-yl)benzoylacetate
3-Aminocrotonate (E)-3-(3-pyridyl)-2-propenylester
3-Phenylpropionaldehyde

NMR: 1.03 (3H, s), 1.91 (2H, m), 2.44 (3H, s), 2.54 (3H, s), 2.74 (2H, m), 4.02 (2H, q), 4.29 (1H, t), 4.88 (2H, d), 6.43 (1H, dt), 6.69 (1H, d), 7.08 (1H, m), 7.16 - 7.28 (4H, m), 7.24 (2H, d), 7.40 (2H, m), 7.54 (2H, d), 7.72 (1H, m), 8.32 (1H, m), 8.49 (1H, dd), 8.62 (1H, d), 9.04 (1H, s)

Example 25

Synthesis of 6-methyl-2-[4-(2-methylimidazo[4,5-c]pyridin-1-yl)phenyl]-4-[(E)-2-phenylethenyl]-1,4-dihydropyridin-3,5-dicarboxylate 3-ethylester, 5-[(E)-3-(3-pyridyl)-2-propenyl]ester (Compound 25)

Starting material

Ethyl 4'-(2-methylimidazo[4,5-c]pyridin-1-yl)benzoylacetate
3-Aminocrotonate (E)-3-(3-pyridyl)-2-propenylester
Cinnamaldehyde

NMR: 1.03 (3H, t), 2.50 (3H, s), 2.56 (3H, s), 4.02 (2H, m), 4.16 (2H, m), 4.85 (1H, dd), 4.87 (1H, d), 4.96 (1H, dd), 6.35 (1H, dd), 6.41 (1H, dd), 6.48 (1H, d), 6.65 (1H, d), 7.02 (1H, d), 7.20 (2H, m), 7.28 (2H, t), 7.37 (2H, d), 7.58 (3H, d), 8.17 (1H, d), 8.45 (1H, q), 8.51 (1H, d), 8.99 (1H, s)

Example 26

Synthesis of 6-methyl-2-[4-(2-methylimidazo[4,5-c]pyridin-1-yl)phenyl]-4-[2-(3-thienyl)ethyl)-1,4-dihydropyridin-3,5-dicarboxylate 3-ethylester, 5-[(E)-3-(3-pyridyl)-2-propenyl]ester (Compound 26)

Starting material

Ethyl 4'-(2-methylimidazo[4,5-c]pyridin-1-yl)benzoylacetate
3-Aminocrotonate (E)-3-(3-pyridyl)-2-propenylester
3-Thiophenepropionaldehyde

NMR: 1.03 (3H, s), 1.92 (2H, m), 2.44 (3H, s), 2.58 (3H, s), 2.76 (2H, m), 4.02 (2H, m), 4.29 (1H, t), 4.86 (2H, d), 6.43 (1H, dt), 6.68 (1H, d), 6.95 (1H, d), 6.98 (1H, m), 7.07 (1H, d), 7.23 (1H, m), 7.27 (1H, t), 7.39 (2H, d), 7.54 (2H, d), 7.72 (1H, m), 8.31 (1H, m), 8.49 (1H, dd), 8.62 (1H, d), 9.04 (1H, s)

Example 27

Synthesis of 4-[(E]-2-(3-furyl)ethenyl]-6-methyl-2-[4-(2-methylimidazo[4,5-c] pyridin-1-yl)phenyl]-1,4-dihydropyridin-3,5-dicarboxylate 3-ethylester, 5-[(E)-3-(3-pyridyl)-2-propenyl]ester (Compound 27)

Starting material

Ethyl 4'-(2-methylimidazo[4,5-c]pyridin-1-yl)benzoylacetate
3-Aminocrotonate (E)-3-(3-pyridyl)-2-propenylester
(E)-3-(3-furyl)propenal

NMR: 1.03 (3H, t), 2.46 (3H, s), 2.58 (3H, s), 4.02 (2H, m), 4.83 (1H, d), 4.86 (2H, m), 6.19 (1H, d), 6.28 (1H, d), 6.42 (1H, dt), 6.66 (1H, d), 7.08 (1H, d), 7.22 - 7.26 (3H, m), 7.33 (1H, s), 7.40 (2H, d), 7.59 (2H, d), 7.65 (1H, m), 8.32 (1H, d), 8.48 (1H, dd), 8.54 (1H, d), 9.05 (1H, s)

Example 28

Synthesis of 4-(2-cyclohexylethyl)-6-methyl-2-[4-(2-methylimidazo[4,5-c]pyridin-1-yl)phenyl]-1,4-dihydropyridin-3,5-dicarboxylate 3-ethylester, 5-[(E)-3-(3-pyridyl)-2-propenyl]ester (Compound 28)

Starting material

Ethyl 4'-(2-methylimidazo[4,5-c]pyridin-1-yl)benzoylacetate
3-Aminocrotonate (E)-3-(3-pyridyl)-2-propenylester
3-Cyclohexylpropanal

NMR: 0.85 - 1.65 (11H, m), 1.03 (3H, t), 2.41 (3H, s), 2.59 (3H, s), 4.01 (2H, m), 4.15 (1H, m), 4.87 (2H, m), 6.46 (1H, dt), 6.70 (1H, d), 7.10 (1H, d), 7.26 (1H, m), 7.40 (2H, d), 7.56 (2H, d), 7.74 (1H, m), 8.35 (1H, d), 8.50 (1H, dd), 8.63 (1H, d), 9.06 (1H, s)

Example 29

Synthesis of 4-cyclohexylidenemethyl-6-methyl-2-[4-(2-methylimidazo[4,5-c]pyridin-1-yl)phenyl]-1,4-dihydropyridin-3,5-dicarboxylate 3-ethylester, 5-[(E)-3-(3-pyridyl)-2-propenyl]ester (Compound 29)

Starting material

Ethyl 4'-(2-methylimidazo[4,5-c]pyridin-1-yl)benzoylacetate
3-Aminocrotonate (E)-3-(3-pyridyl)-2-propenylester
Cyclohexylidenacetoaldehyde

NMR: 1.05 (3H, t), 1.52 (4H, m), 2.04 (2H, m), 2.40 (2H, m), 2.45 (3H, s), 2.55 (3H, s), 4.00 (2H, m), 4.85 (1H, m), 4.89 (1H, d), 5.13 (1H, d), 6.44 (1H, dt), 6.66 (1H, d), 7.01 (1H, d), 7.28 (1H, m), 7.32 (2H, d), 7.58 (2H, d), 7.73 (1H, d), 8.15 (1H, d), 8.48 (1H, q), 8.61 (1H, d), 8.97 (1H, s)

Example 30

Synthesis of 6-methyl-2-[4-(4-methylpiperadin-1-yl)phenyl]-1,4-dihydropyridin-4-[(E)-2-phenylethenyl]-3,5-dicarboxylate 3-ethylester, 5-[(E)-3-(3-pyridyl)-2-propenyl]ester (Compound 30)

Starting material

Ethyl 4'-(4-methylpiperadin-1-yl)benzoylacetate
3-Aminocrotonate (E)-3-(3-pyridyl)-2-propenylester
Cinnamaldehyde

NMR: 0.99 (3H, t), 2.36 (3H, s), 2.40 (3H, s), 2.57 (4H, m), 3.26 (4H, m), 3.98 (2H, q), 4.77 (1H, d), 4.78 (1H, dd), 4.94 (1H, dd), 6.31 (1H, dd), 6.38 (1H, dd), 6.40 (1H, d), 6.63 (1H, d), 6.91 (2H, d), 7.19 (2H, m), 7.24 (2H,

m), 7.25 (4H, m), 7.34 (2H, d), 7.58 (1H, d), 8.45 (1H, q), 8.51 (1H, d)

Example 31

Synthesis of 6-methyl-2-(4-methylthiophenyl)-1,4-dihydropyridin-4-[(E)-2-phenylethenyl]-3,5-dicarboxylate 3-ethylester, 5-[(E)-3-(3-pyridyl)-2-propenyl]ester (Compound 31)

Starting material

Ethyl 4'-methylthiobenzoylacetate
3-Aminocrotonate (E)-3-(3-pyridyl)-2-propenylester
Cinnamaldehyde

NMR: 0.98 (3H, t), 2.40 (3H, s), 2.50 (3H, s), 3.97 (2H, q), 4.79 (1H, d), 4.80 (1H, dd), 4.93 (1H, dd), 6.31 (1H, dd), 6.38 (1H, dd), 6.40 (1H, d), 6.63 (1H, d), 6.91 (2H, d), 7.19 (2H, d), 7.23 (2H, m), 7.26 (4H, m), 7.34 (2H, d), 7.59 (1H, d), 8.45 (1H, q), 8.51 (1H, d)

Example 32

Synthesis of 2-[4-(1H-benzotriazol-1-yl)phenyl]-6-methyl-4-[(E)-2-phenylethenyl]-1,4-dihydropyridin-3,5-dicarboxylate 3-ethylester, 5-[(E)-3-(3-pyridyl)-2-propenyl]ester (Compound 32)

Starting material

Ethyl 4'-(1H-benzotriazol-1-yl)benzoylacetate
3-Aminocrotonate (E)-3-(3-pyridyl)-2-propenylester
Cinnamaldehyde

NMR: 1.00 (3H, t), 2.47 (3H, s), 4.00 (2H, m), 4.79 (1H, dd), 4.85 (1H, d), 4.95 (1H, dd), 6.35 (1H, dd), 6.41 (1H, dd), 6.45 (1H, d), 6.64 (1H, d), 7.20 (2H, m), 7.28 (2H, m), 7.38 (2H, d), 7.46 (1H, q), 7.56 (2H, m), 7.59 (2H, d), 7.77 (1H, d), 7.85 (2H, d), 8.16 (1H, d), 8.45 (1H, q), 8.51 (1H, d)

Example 33

Synthesis of 2-[4-(1-imidazolyl)phenyl]-6-methyl-4-[(E)-2-phenylethenyl]-1,4-dihydropyridin-3,5-dicarboxylate 3-ethylester, 5-[(E)-3-(3-pyridyl)-2-propenyl)ester (Compound 33)

Starting material

Ethyl 4'-(1-imidazolyl)benzoylacetate
3-Aminocrotonate (E)-3-(3-pyridyl)-2-propenylester
Cinnamaldehyde

NMR: 1.02 (3H, t), 2.48 (3H, s), 3.99 (2H, m), 4.79 (1H, dd), 4.82 (1H, d), 4.94 (1H, dd), 6.33 (1H, dd), 6.39 (1H, dt), 6.42 (1H, d), 6.64 (1H, d), 7.04 (1H, s), 7.17 (1H, s), 7.20 (2H, d), 7.27 (4H, m), 7.34 (2H, d), 7.37 (1H, s), 7.42 (2H, d), 7.57 (1H, d), 8.45 (1H, q), 8.51 (1H, d)

Example 34

Synthesis of 6-methyl-2-(4-morpholinophenyl)-4-[(E)-2-phenylethenyl]-1,4-dihydropyridin-3,5-dicarboxylate 3-ethylester, 5-[(E)-3-(3-pyridyl)-2-propenyl]ester (Compound 34)

Starting material

Ethyl 4'-morpholinobenzoylacetate
3-Aminocrotonate (E)-3-(3-pyridyl)-2-propenylester
Cinnamaldehyde

NMR: 1.00 (3H, t), 2.40 (3H, s), 3.20 (4H, m), 3.86 (4H, m), 3.98 (2H, m), 4.77 (1H, dd), 4.78 (1H, d), 4.93 (1H, dd), 6.32 (1H, dd), 6.36 (1H, d), 6.39 (1H, m), 6.63 (1H, d), 6.89 (2H, d), 7.18 (2H, m), 7.26 (3H, m), 7.35 (2H, d), 7.36 (1H, m), 7.58 (1H, d), 8.45 (1H, q), 8.50 (1H, d)

Example 35

Synthesis of 2-[4-(1-indolyl)phenyl]-6-methyl-4-[(E)-2-phenylethenyl]-1,4-dihydropyridin-3,5-dicarboxylate 3-ethylester, 5-[(E)-3-(3-pyridyl)-2-propenyl]ester (Compound 35)

Starting material

Ethyl 4'-(1-indolyl)benzoylacetate
3-Aminocrotonate (E)-3-(3-pyridyl)-2-propenylester
Cinnamaldehyde

NMR: 1.00 (3H, t), 2.44 (3H, s), 4.00 (2H, m), 4.79 (1H, dd), 4.84 (1H, d), 4.94 (1H, dd), 6.35 (1H, dd), 6.40 (1H, dd), 6.44 (1H, d), 6.64 (1H, d), 6.71 (1H, d), 7.20 (4H, m), 7.27 (3H, m), 7.37 (3H, m), 7.50 (2H, d), 7.56 (2H, d), 7.59 (2H, m), 7.70 (1H, d), 8.44 (1H, q), 8.51 (1H, d)

Example 36

Synthesis of 2-[3-(1-imidazolyl)phenyl]-6-methyl-4-[(E)-2-phenylethenyl]-1,4-dihydropyridin-3,5-dicarboxylate 3-ethylester, 5-[(E)-3-(3-pyridyl)-2-propenyl]ester (Compound 36)

Starting material

Ethyl 3'-(1-imidazolyl)benzoylacetate
3-Aminocrotonate (E)-3-(3-pyridyl)-2-propenylester
Cinnamaldehyde

NMR: 0.96 (3H, t), 2.45 (3H, s), 3.96 (2H, m), 4.81 (1H, dd), 4.83 (1H, d), 4.93 (1H, dd), 6.35 (1H, dd), 6.37 (1H, dt), 6.42 (1H, d), 6.62 (1H, d), 7.14 (1H, s), 7.19 (1H, s), 7.20 (2H, d), 7.27 (4H, m), 7.34 (2H, d), 7.37 (1H, s), 7.42 (2H, d), 7.68 (1H, s), 8.44 (1H, q), 8.48 (1H, d)

Example 37

Synthesis of 2-[3-(1-imidazolyl)phenyl]-6-methyl-4-pentyl-1,4-dihydropyridin-3,5-dicarboxylate 3-ethylester, 5-[(E)-3-(3-pyridyl)-2-propenyl]ester (Compound 37)

Starting material

Ethyl 3'-(1-imidazolyl)benzoylacetate
3-Aminocrotonate (E)-3-(3-pyridyl)-2-propenylester
Capronaldehyde

NMR: 0.84 (3H, t), 0.95 (3H, t), 1.24 - 1.50 (8H, m), 2.39 (3H, s), 3.94 (2H, m), 4.11 (1H, t), 4.84 (2H, m), 6.42 (1H, dt), 6.66 (1H, d), 7.11 (1H, S), 7.22 (1H, s), 7.26 (3H, m), 7.32 (2H, m), 7.49 (1H, t), 7.63 (1H, s), 7.72 (1H, d), 8.45 (1H, dd), 8.57 (1H, d)

Example 38

Synthesis of 4-cyclohexyl-2-[4-(1-imidazolyl)phenyl]-6-methyl-1,4-dihydropyridin-3,5-dicarboxylate 3-methylester, 5-[(E)-3-(3-pyridyl)-2-propenyl]ester (Compound 38)

Starting material

Methyl 4'-(1-imidazolyl)benzoylacetate
3-Aminocrotonate (E)-3-(3-pyridyl)-2-propenylester

Cyclohexylaldehyde

NMR: 1.06 - 1.72 (11H, m), 2.44 (3H, s), 3.54 (3H, s), 4.09 (1H, d), 4.87 (2H, m), 6.45 (1H, dt), 6.70 (1H, d), 7.20 (1H, s), 7.28 (1H, q), 7.30 (1H, s), 7.41 (2H, m), 7.44 (2H, d), 7.69 (1H, s), 7.73 (1H, d), 8.49 (1H, dd), 8.62 (1H, d)

Example 39

Synthesis of 2-[4-(1-imidazolyl)phenyl]-6-methyl-1,4-dihydropyridin-4-[(E)-2-phenylethenyl]-3,5-dicarboxylate 3-methylester, 5-[(E)-3-(3-pyridyl)-2-propenyl]ester (Compound 39)

Starting material

Methyl 4'-(1-imidazolyl)benzoylacetate
3-aminocrotonate (E)-3-(3-pyridyl)-2-propenylester
Cinnamaldehyde

NMR: 2.48 (3H, s), 3.56 (3H, s), 4.80 (1H, dd), 4.81 (1H, d), 4.96 (1H, dd), 6.35 (1H, dd), 6.41 (1H, d), 6.43 (1H, m), 6.63 (1H, d), 6.97 (1H, s), 7.17 (1H, s), 7.20 (2H, m), 7.28 (2H, m), 7.35 (2H, d), 7.36 (2H, m), 7.43 (2H, d), 7.46 (1H, s), 7.59 (1H, d), 8.46 (1H, q), 8.52 (1H, d)

Example 40

Synthesis of 2-[4-(1-imidazolyl)phenyl]-6-methyl-4-phenylethynyl-1,4-dihydropyridin-3,5-dicarboxylate 3-methylester, 5-[(E)-3-(3-pyridyl)-2-propenyl]ester (Compound 40)

Starting material

Methyl 4'-(1-imidazolyl)benzoylacetate
3-aminocrotonate (E)-3-(3-pyridyl)-2-propenylester
Phenylpropargylaldehyde

NMR: 2.49 (3H, s), 3.62 (3H, s), 4.84 (1H, dd), 4.87 (1H, d), 5.03 (1H, dd), 5.19 (1H, s), 6.45 (1H, dt), 6.74 (1H, d), 7.17 (1H, s), 7.21 (2H, m), 7.24 (1H, s), 7.37 (1H, s), 7.38 (2H, d), 7.39 (2H, m), 7.40 (2H, d), 7.45 (2H, d), 7.62 (1H, d), 8.46 (1H, t), 8.55 (1H, d)

Example 41

Synthesis of 6-methyl-2-[4-(2-methylimidazo[4,5-c]pyridin-1-yl)phenyl]-4-[(E)-2-(3-thienyl)ethenyl-1,4-dihydropyridin-3,5-dicarboxylate 3-methylester, 5-[(E)-3-(3-pyridyl)-2-propenyl]ester (Compound 41)

Starting material

Methyl 4'-(2-methylimidazo[4,5-c]pyridin-1-yl)benzoylacetate
3-Aminocrotonate (E)-3-(3-pyridyl)-2-propenylester
(E)-3-(3-thienyl)propenal

NMR: 2.50 (3H, s), 2.57 (3H, s), 3.58 (3H, s), 4.80 (1H, dd), 4.82 (1H, d), 4.8 (1H, dd), 6.21 (1H, dd), 6.42 (1H, dt), 6.45 (1H, d), 6.65 (1H, d), 7.05 (1H, d), 7.10 (1H, m), 7.21 - 7.26 (3H, m), 7.35 (2H, d), 7.58 (2H, d), 7.62 (1H, m), 8.20 (1H, d), 8.46 (1H, dd), 8.51 (1H, d), 9.00 (1H, s)

Example 42

Synthesis of 6-methyl-2-[4-(2-methylimidazo[4,5-c]pyridin-1-yl)phenyl]-4-[(E)-2-phenylethenyl]-1,4-dihydropyridin-3,5-dicarboxylate 3-methylester, 5-[(E)-3-(3-pyridyl)-2-propenyl]ester (Compound 42)

Starting material

Methyl 4'-(2-methylimidazo[4,5-c]pyridin-1-yl)benzoylacetate
3-Aminocrotonate (E)-3-(3-pyridyl)-2-propenylester
Cinnamaldehyde

NMR: 2.48 (3H, s), 2.58 (3H, s), 3.59 (3H, s), 4.85 (1H, d), 4.89 (1H, m), 6.40 (1H, dt), 6.42 (1H, d), 6.66 (1H, d), 7.08 (1H, d), 7.21 (2H, m), 7.28 (2H, t), 7.36 (2H, d), 7.38 (2H, d), 7.60 (3H, d), 8.27 (1H, d), 8.46 (1H, d), 8.53 (1H, m), 9.03 (1H, s)

Example 43

Synthesis of 2-[4-(1-imidazolyl)phenyl]-6-methyl-4-[(E)-2-phenylethenyl]-1,4-dihydropyridin-3,5-dicarboxylate 3-ethylester, 5-[3-(3-pyridyl)propyl]ester (Compound 43)

Starting material

Ethyl 4'-(1-imidazolyl)benzoylacetate
3-Aminocrotonate 3-(3-pyridyl)propylester
Cinnamaldehyde

NMR: 1.03 (3H, t), 2.02 (2H, m), 2.46 (3H, s), 2.74 (2H, t), 4.00 (2H, m), 4.16 (1H, m), 4.24 (1H, m), 4.80 (1H, d), 6.32 (1H, dd), 6.41 (1H, d), 7.15 (1H, dd), 7.19 (1H, s), 7.22 (1H, d), 7.28 (5H, m), 7.36 (2H, d), 7.37 (1H, s), 7.40 (2H, d), 7.50 (1H, s), 8.41 (1H, d), 8.42 (1H, d)

Example 44

Synthesis of 2-[4-(1-imidazolyl)phenyl]-6-methyl-4-pentyl-1,4-dihydropyridin-3,5-dicarboxylate 3-ethylester, 5-[3-(3-pyridyl)propyl]-ester (Compound 44)

Starting material

Ethyl 4'-(1-imidazolyl)benzoylacetate
3-Aminocrotonate 3-(3-pyridyl)propylester
Capronaldehyde

NMR: 0.88 (3H, t), 1.03 (3H, t), 1.22 - 1.53 (8H, m), 2.04 (2H, m), 2.41 (3H, s), 2.78 (2H, t), 3.99 (2H, m), 4.11 (1H, t), 4.20 (2H, m), 7.18 (1H, s), 7.22 (1H, m), 7.28 (1H, s), 7.36 (2H, d), 7.41 (2H, d), 7.51 (1H, s), 7.53 (1H, m), 8.46 (1H, q), 8.49 (1H, d)

Example 45

Synthesis of 4-(3-cyclohexylpropyl)-6-methyl-2-[4-(2-methylimidazo[4,5-c]pyridin-1-yl)phenyl]-1,4-dihydropyridin-3,5-dicarboxylate 3-ethylester, 5-(3-nicotinoylpropyl)ester (Compound 45)

Starting material

Ethyl 4'-(2-methylimidazo[4,5-c]pyridin-1-yl)benzoylacetate
3-Aminocrotonate 3-(nicotinoyl)propylester
4-Cyclohexylbutylaldehyde

NMR: 0.84 - 1.65 (11H, m), 1.03 (3H, t), 2.20 (2H, m), 2.38 (3H, s), 2.59 (3H, s), 3.16 (2H, t), 4.01 (2H, m), 4.10 (1H, t), 4.30 (2H, m), 7.10 (1H, d), 7.40 (2H, d), 7.44 (1H, dd), 7.56 (2H, d), 8.27 (1H, m), 8.34 (1H, d), 8.79 (1H, dd), 9.05 (1H, s), 9.20 (1H, d)

Example 46

Synthesis of 4-cyclohexylidenemethyl-6-methyl-2-[4-(2-methylimidazo[4,5-c]pyridin-1-yl)phenyl]-1,4-dihydropyridin-3,5-dicarboxylate 3-ethylester 5-(3-nicotinoylpropyl)ester (Compound 46)

Starting material

Ethyl 4'-(2-methylimidazo[4,5-c]pyridin-1-yl)benzoylacetate
3-Aminocrotonate 3-(nicotinoyl)propylester
Cyclohexylidenacetoaldehyde

NMR: 1.04 (3H, t), 1.51 (4H, m), 2.01 (2H, m), 2.19 (2H, m), 2.39 (2H, m), 2.37 (3H, s), 2.58 (3H, s), 3.14 (2H, m), 3.99 (2H, m), 4.26 (1H, m), 4.32 (1H, m), 4.83 (1H, d), 5.08 (1H, d), 7.09 (1H, d), 7.39 (2H, d), 7.44 (1H, dd), 7.59 (2H, d), 8.27 (1H, m), 8.32 (1H, d), 8.79 (1H, dd), 9.04 (1H, s), 9.19 (1H, d)

Example 47

Synthesis of 6-methyl-2-[4-(2-methylimidazo[4,5-c]pyridin-1-yl)phenyl]-4-[(E)-2-phenylethenyl]-1,4-dihydropyridin-3,5-dicarboxylate 3-ethylester, 5-(3-nicotinoylpropyl)ester (Compound 47)

Starting material

Ethyl 4'-(2-methylimidazo[4,5-c]pyridin-1-yl)benzoylacetate
3-Aminocrotonate 3-(nicotinoyl)propylester
Cinnamaldehyde

NMR: 1.00 (3H, t), 2.18 (2H, m), 2.50 (3H, s), 2.53 (3H, s), 3.09 (2H, t), 4.00 (2H, m), 4.24 (1H, m), 4.37 (1H, m), 4.81 (1H, d), 6.33 (1H, dd), 6.42 (1H, d), 6.97 (1H, d), 7.15 (1H, t), 7.23 (2H, t), 7.29 (2H, m), 7.34 (2H, d), 7.51 (1H, s), 7.57 (2H, d), 8.04 (1H, d), 8.06 (1H, m), 8.71 (1H, dd), 8.94 (1H, s), 9.07 (1H, d)

Example 48

Synthesis of 4-cyclohexylidenemethyl-6-methyl-2-[4-(1-benzimidazolyl)phenyl]-1,4-dihydropyridin-3,5-dicarboxylate 3-ethylester, 5-(3-nicotinoylpropyl)ester (Compound 48)

Starting material

Ethyl 4'-(benzimidazoyl-1-yl)benzoylacetate
3-Aminocrotonate 3-(nicotinoyl)propylester
Cyclohexylidenacetoaldehyde

NMR: 1.03 (3H, t), 1.51 (4H, m), 2.02 (2H, m), 2.19 (2H, m), 2.39 (2H, m), 2.44 (3H, s), 3.14 (2H, m), 3.98 (2H, m), 4.26 (1H, m), 4.32 (1H, m), 4.82 (1H, d), 5.10 (1H, d), 7.35 (2H, m), 7.43 (1H, m), 7.48 (2H, d), 7.52 (1H, m), 7.53 (2H, d), 7.73 (1H, s), 7.86 (1H, m), 8.26 (1H, m), 8.79 (1H, dd), 9.19 (1H, d)

The structures of the substituents of the compounds (Compound 1 to Compound 48) obtained in the above Examples are shown in Table 1 to Table 7.

Table 1

| Compound | $R_3$ | $R_2$ | $R_1$ | $R_4$ |
|---|---|---|---|---|
| Compound 1 | 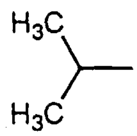 | $-CH_2CH_3$ | 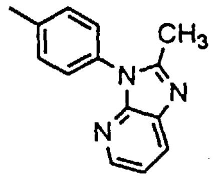 | 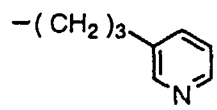 |
| Compound 2 | 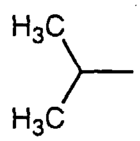 | $-CH_2CH_3$ | 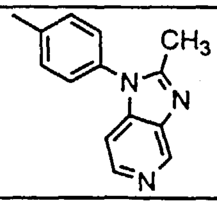 | 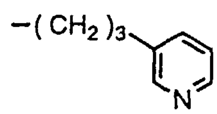 |
| Compound 3 | $CH_3(CH_2)_4-$ | $-CH_2CH_3$ | 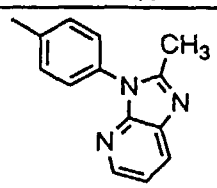 | 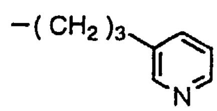 |
| Compound 4 | $CH_3(CH_2)_4-$ | $-CH_2CH_3$ | 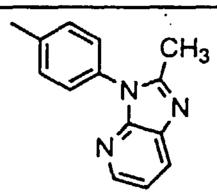 | 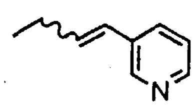 |
| Compound 5 | $CH_3(CH_2)_4-$ | $-CH_2CH_3$ | 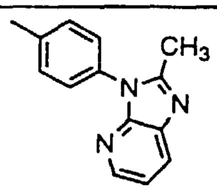 | 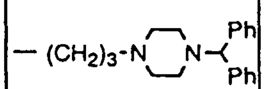 |
| Compound 6 | $CH_3(CH_2)_3-$ | $-CH_2CH_3$ | 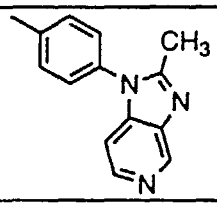 | 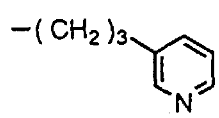 |
| Compound 7 | $CH_3(CH_2)_4-$ | $-CH_2CH_3$ | 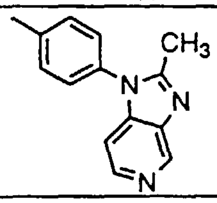 | 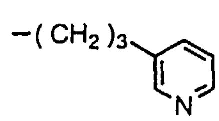 |

Table 2

| Compound | $R_3$ | $R_2$ | $R_1$ | $R_4$ |
|---|---|---|---|---|
| Compound 8 | 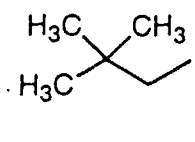 | $-CH_2CH_3$ | 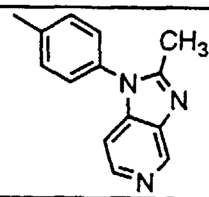 | 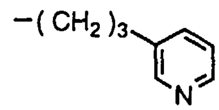 |
| Compound 9 | 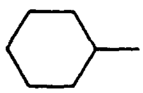 | $-CH_2CH_3$ | 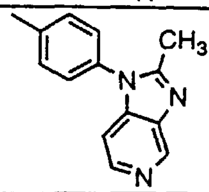 | 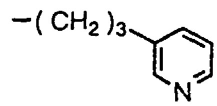 |
| Compound 10 | 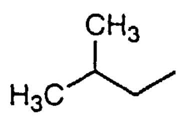 | $-CH_2CH_3$ | 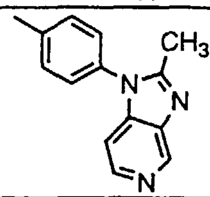 | 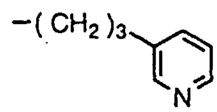 |
| Compound 11 | 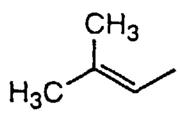 | $-CH_2CH_3$ | 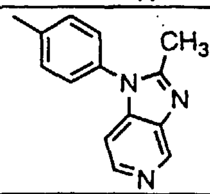 | 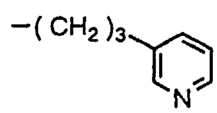 |
| Compound 12 | 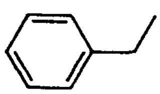 | $-CH_2CH_3$ | 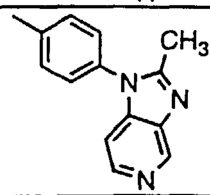 | 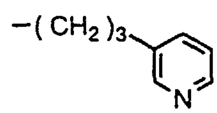 |
| Compound 13 | 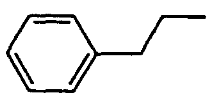 | $-CH_2CH_3$ | 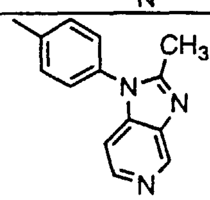 | 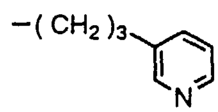 |
| Compound 14 | 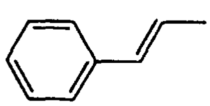 | $-CH_2CH_3$ | 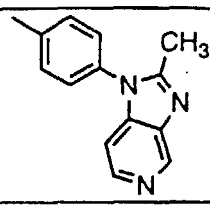 | 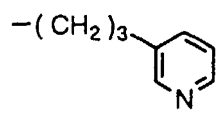 |

Table 3

| Compound | $R_3$ | $R_2$ | $R_1$ | $R_4$ |
|---|---|---|---|---|
| Compound 15 | $CH_3(CH_2)_4-$ | $-CH_2CH_3$ | 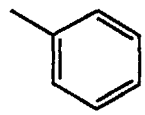 | 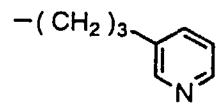 |
| Compound 16 | $CH_3(CH_2)_4-$ | $-CH_2CH_3$ | 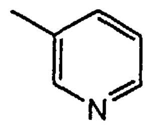 | 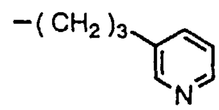 |
| Compound 17 | $CH_3(CH_2)_4-$ | $-CH_2CH_3$ | 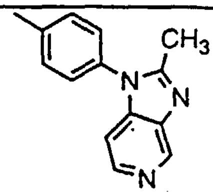 | 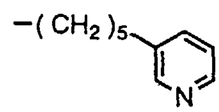 |
| Compound 18 | 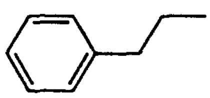 | $-CH_2CH_3$ | 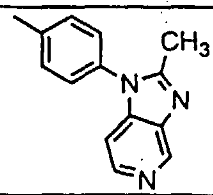 | 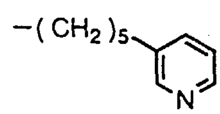 |
| Compound 19 | $CH_3(CH_2)_4-$ | $-CH_2CH_3$ | 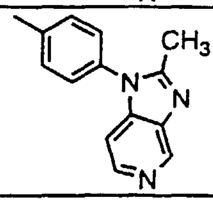 | 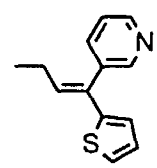 |
| Compound 20 | 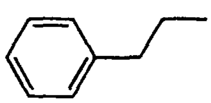 | $-CH_2CH_3$ | 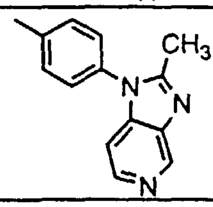 | 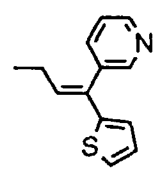 |
| Compound 21 | 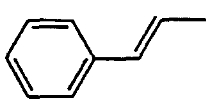 | $-CH_2CH_3$ | 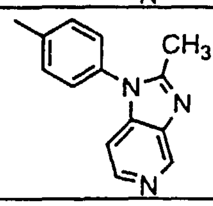 | 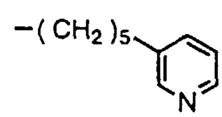 |

Table 4

| Compound | $R_3$ | $R_2$ | $R_1$ | $R_4$ |
|---|---|---|---|---|
| Compound 22 | 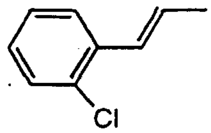 | $-CH_2CH_3$ | 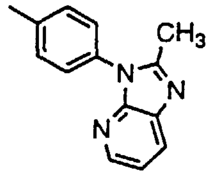 | 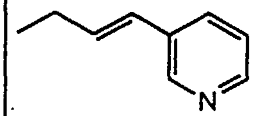 |
| Compound 23 | $CH_3(CH_2)_4-$ | $-CH_2CH_3$ | 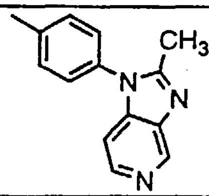 | 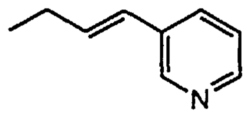 |
| Compound 24 | 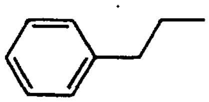 | $-CH_2CH_3$ | 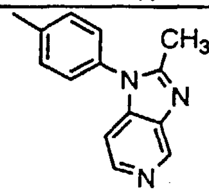 | 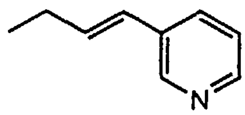 |
| Compound 25 | 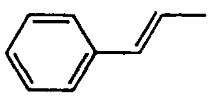 | $-CH_2CH_3$ | 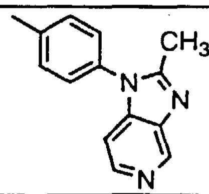 | 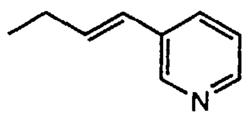 |
| Compound 26 | 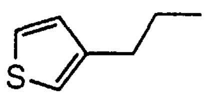 | $-CH_2CH_3$ | 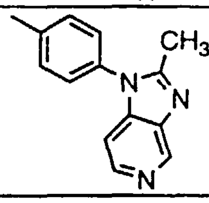 | 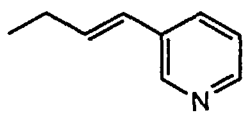 |
| Compound 27 | 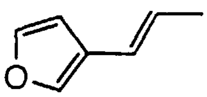 | $-CH_2CH_3$ | 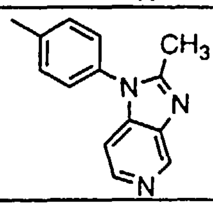 | 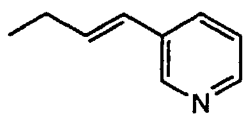 |
| Compound 28 | 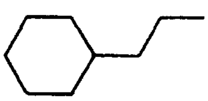 | $-CH_2CH_3$ | 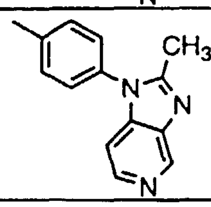 | 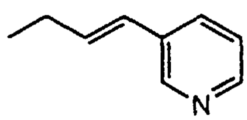 |

Table 5

| Compound | $R_3$ | $R_2$ | $R_1$ | $R_4$ |
|---|---|---|---|---|
| Compound 29 | 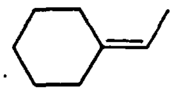 | $-CH_2CH_3$ | 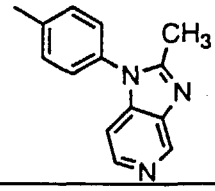 | 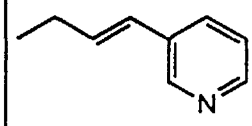 |
| Compound 30 | 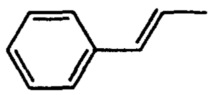 | $-CH_2CH_3$ | 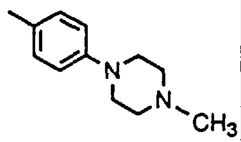 | 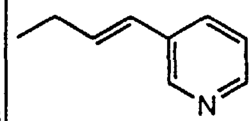 |
| Compound 31 | 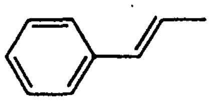 | $-CH_2CH_3$ | 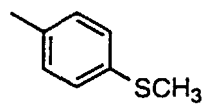 | 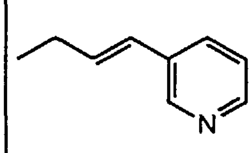 |
| Compound 32 | 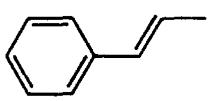 | $-CH_2CH_3$ | 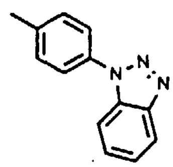 | 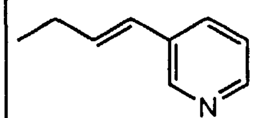 |
| Compound 33 | 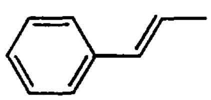 | $-CH_2CH_3$ | 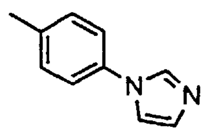 | 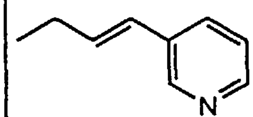 |
| Compound 34 | 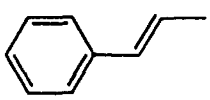 | $-CH_2CH_3$ | 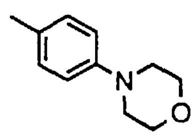 | 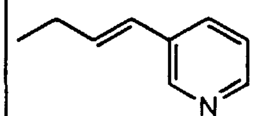 |
| Compound 35 | 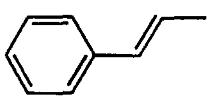 | $-CH_2CH_3$ | 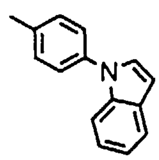 | 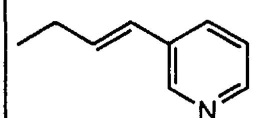 |

Table 6

| Compound | $R_3$ | $R_2$ | $R_1$ | $R_4$ |
|---|---|---|---|---|
| Compound 36 | 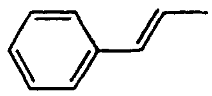 | $-CH_2CH_3$ | 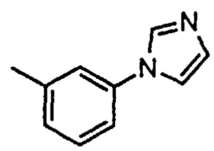 | 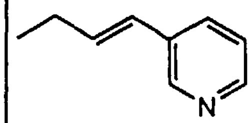 |
| Compound 37 | $CH_3(CH_2)_4-$ | $-CH_2CH_3$ | 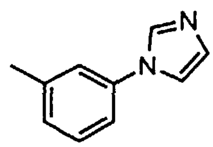 | 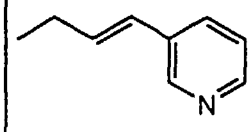 |
| Compound 38 | 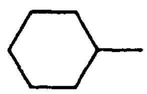 | $-CH_3$ | 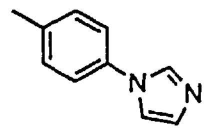 | 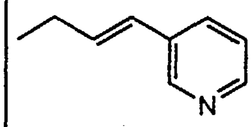 |
| Compound 39 | 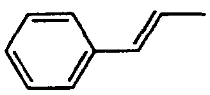 | $-CH_3$ | 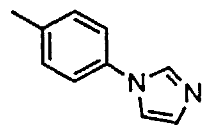 | 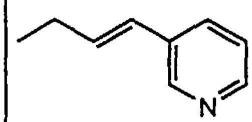 |
| Compound 40 | 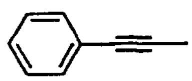 | $-CH_3$ | 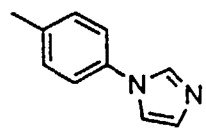 | 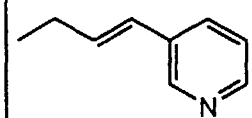 |
| Compound 41 | 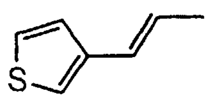 | $-CH_3$ | 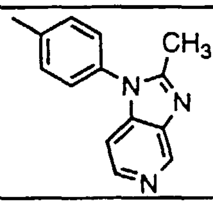 | 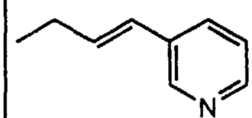 |
| Compound 42 | 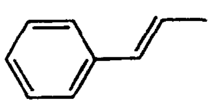 | $-CH_3$ | 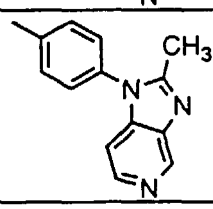 | 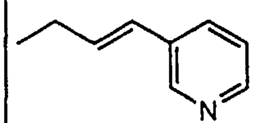 |

Table 7

| Compound | $R_3$ | $R_2$ | $R_1$ | $R_4$ |
|---|---|---|---|---|
| Compound 43 | 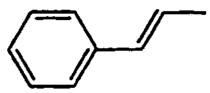 | $-CH_2CH_3$ | 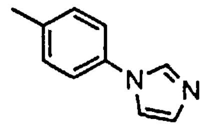 | 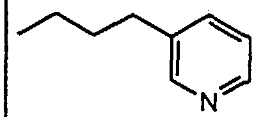 |
| Compound 44 | $CH_3(CH_2)_4-$ | $-CH_2CH_3$ | 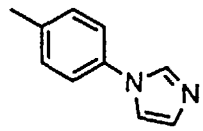 | 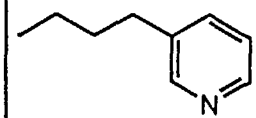 |
| Compound 45 | 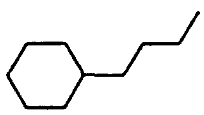 | $-CH_2CH_3$ | 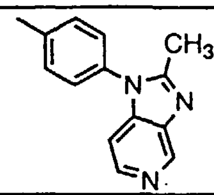 | 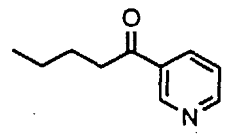 |
| Compound 46 | 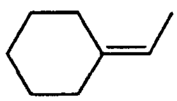 | $-CH_2CH_3$ | 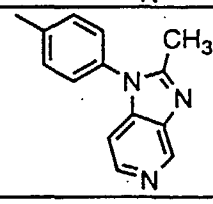 | 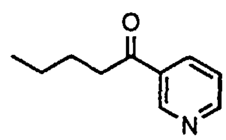 |
| Compound 47 | 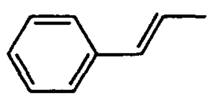 | $-CH_2CH_3$ | 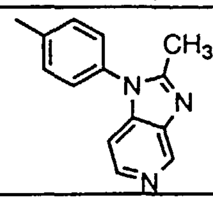 | 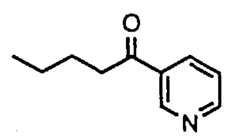 |
| Compound 48 | 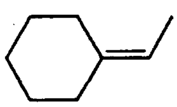 | $-CH_2CH_3$ | 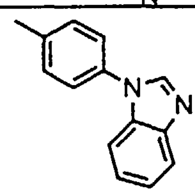 | 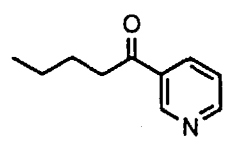 |

Preparation Examples

Example 49 (Preparation of Tablets]

| | |
|---|---|
| Present Compound (Compound 1) | 25 g |
| Lactose | 62 g |
| Corn starch | 40 g |
| Hydroxypropylcellulose | 2 g |
| Magnesium stearate | 1 g |

The present compound (i.e., Compound 1), lactose, and corn starch were mixed until becoming homogeneous, then a 5 w/v% ethanol solution of hydroxypropylcellulose was added and the mixture kneaded and granulated. The granules were passed through a 16 mesh sieve to standardize their size, then form to tablets by an ordinary method to obtain tablets of a weight per tablet of 130 mg, a diameter of 7 mm, and a content of the main drug of 25 mg.

Test Examples

Test Example 1

In Vitro Test of Action Suppressing Proliferation of Cancer Cells combined administration of Doxorubicin

Human rhinopharynx cancer derived KB cells (i.e., sensitive cells) and their multi-drug resistant clone VJ-300 cells (i.e., resistant cells) were used as the test cells. As the incubation solutions, use was made of 10% fetal calf serum (Flow Laboratories) and Eagle MEM medium (Nissui Seiyaku) containing 0.292 mg/ml of L-glutamine (Flow Laboratories). The tests of the action for overcoming resistance to anti-cancer drugs or action for increasing the effect of the anti-cancer drug with the combined administration of the anti-cancer drug doxorubicin (i.e., adriamycin, ADM) and the test compound were performed as follows:

The test cells were suspended in incubation solutions and adjusted to a cell density of about 200 cells/ml. The cell suspensions were injected in 2 ml amounts into petri dishes and were incubated in a $CO_2$ gas incubator (5% $CO_2$, 95% air) at 37°C for 24 hours. Then, 5 μl of a predetermined concentration of the aqueous ADM solution and a predetermined concentration of a DMSO solution of the test compound were added, then the incubation was continued for a further 7 days. After the end of the incubation, the result was immobilized with methanol, Giemsa stained and measured for the number of colonies per dish. The results were used to prepare a volume-reaction curve. From this, the ADM concentration of the 50% cell survival rate ($LD_{50}$) was calculated and the effect for overcoming resistance to anti-cancer drugs and the effect for increasing the effect of anti-cancer drugs were judged. The results are shown in Table 8 to Table 10 using the concentration of $LD_{50}$ of ADM in the ADM alone group by KB cells as the resistance degree 1 and calculating the resistances of the following $LD_{50}$ concentrations as relative ratios. In the tables, "ADM alone (control)" shows the group administered just ADM, "ADM + Compound 1" shows the group combined administration of the ADM and the Compound 1 (1 μg/ml) and, similarly below, "ADM + Compound 48" shows the group combined administration of the ADM and the Compound 48 (1 μg/ml).

Table 8

| | Degree of resistance for ADM | |
|---|---|---|
| | KB | VJ-300 |
| ADM alone (control) | 1.0 | 26 - 51 |
| ADM + Compound 1 | 0.7 | 2.4 |

Table 8 (continued)

| | Degree of resistance for ADM | |
|---|---|---|
| | KB | VJ-300 |
| ADM + Compound 2 | 0.8 | 2.7 |
| ADM + Compound 3 | 0.5 | 1.2 |
| ADM + Compound 4 | 0.8 | 1.2 |
| ADM + Compound 5 | 0.9 | 4.6 |
| ADM + Compound 6 | 1.0 | 2.8 |
| ADM + Compound 7 | 1.0 | 2.8 |
| ADM + Compound 8 | 0.4 | 1.3 |
| ADM + Compound 9 | 0.3 | 2.9 |
| ADM + Compound 10 | 0.5 | 1.4 |
| ADM + Compound 11 | 0.9 | 3.2 |
| ADM + Compound 12 | 0.5 | 6.5 |
| ADM + Compound 13 | 1.0 | 2.6 |
| ADM + Compound 14 | 0.6 | 1.3 |
| ADM + Compound 15 | 0.8 | 3.2 |
| ADM + Compound 16 | 0.9 | 3.0 |
| ADM + Compound 17 | 0.6 | 1.6 |
| ADM + Compound 18 | 0.5 | 1.4 |
| ADM + Compound 19 | 0.7 | 1.2 |
| ADM + Compound 20 | 0.8 | 1.6 |

Table 9

| | Degree of resistance for ADM | |
|---|---|---|
| | KB | VJ-300 |
| ADM alone (control) | 1.0 | 26 - 51 |
| ADM + Compound 21 | 1.1 | 1.1 |
| ADM + Compound 22 | 0.6 | 1.7 |
| ADM + Compound 23 | 1.0 | 1.2 |
| ADM + Compound 24 | 1.1 | 1.1 |
| ADM + Compound 25 | 0.7 | 1.1 |
| ADM + Compound 26 | 1.0 | 1.8 |
| ADM + Compound 27 | 0.8 | 8.7 |
| ADM + Compound 28 | 1.1 | 1.7 |
| ADM + Compound 29 | 1.1 | 1.2 |
| ADM + Compound 30 | 1.0 | 1.0 |

Table 9 (continued)

| | Degree of resistance for ADM | |
|---|---|---|
| | KB | VJ-300 |
| ADM + Compound 31 | 1.0 | 2.6 |
| ADM + Compound 32 | 1.0 | 1.4 |
| ADM + Compound 33 | 0.8 | 1.4 |
| ADM + Compound 34 | 1.2 | 3.0 |
| ADM + Compound 35 | 1.1 | 3.0 |
| ADM + Compound 36 | 0.8 | 1.0 |
| ADM + Compound 37 | 1.1 | 2.5 |
| ADM + Compound 38 | 1.1 | 2.4 |
| ADM + Compound 39 | 1.0 | 1.2 |
| ADM + Compound 40 | 1.0 | 2.5 |

Table 10

| | Degree of resistance for ADM | |
|---|---|---|
| | KB | VJ-300 |
| ADM alone (control) | 1.0 | 26 - 51 |
| ADM + Compound 41 | 0.8 | 7.8 |
| ADM + Compound 42 | 0.8 | 2.1 |
| ADM + Compound 43 | 0.9 | 1.4 |
| ADM + Compound 44 | 0.6 | 1.4 |
| ADM + Compound 45 | 0.9 | 2.6 |
| ADM + Compound 46 | 1.0 | 2.5 |
| ADM + Compound 47 | 1.1 | 1.2 |
| ADM + Compound 48 | 1.1 | 1.7 |

Test Example 2

In Vitro Test of Action Suppressing Proliferation of Cancer Cells by Combined Administration of Vincristine

The same method as in Test Example 1 was used to test the action of vincristine (VCR) on anti-cancer drugs, prepare a volume-reaction curve, and calculate the resistances. The results are shown in Table 11. In Table 11, "VCR alone (control)" shows the group administered with just VCR, "VCR + Compound 2" shows the group combined administration of VCR and the Compound 2 (1 μg/ml), and, the following similarly, "VCR + Compound 15" indicates the group combined administration of VCR and the Compound 15 (1 μg/ml). Further, "VCR+verapamil" shows the group combined administration of VCR and verapamil (1 μg/ml].

Table 11

| | Degree of resistance for VCR | |
|---|---|---|
| | KB | VJ-300 |
| VCR alone (control) | 1.0 | 483 - 952 |
| VCR + Compound 2 | 0.4 | 9.6 |
| VCR + Compound 3 | 0.8 | 7.1 |
| VCR + Compound 15 | 1.2 | 6.5 |
| VCR + Verapamil | 0.5 | 36.9 |

Test Example 3

In Vivo Test of Action Suppressing Proliferation of Cancer Cells by Combined Administration of Anti-cancer Drug

Effect of Overcoming Resistance to an Anti-cancer Drug for Vincristine (VCR) Resistant Murine Leukemia Cell Bearing Mice

$10^6$ VCR resistant murine leukemia (P388/VCR) cells were transplanted intraperitoneally into groups of six $CDF_1$ male mice, then the compound of the present invention (100 mg/kg) and VCR (100 μg/kg) were administered intraperitoneally for 5 days. The number of survival days were found and the life prolonging rate (T/C) with respect to the controls was found. The effect in overcoming resistance to an anti-cancer drug (T/V) was found by the following formula. The results are shown in Table 12 to Table 14. In the tables, "control" shows the group not administered anything, "VCR alone" shows the group administered just VCR (100 μg/kg), "VCR + Compound 1" shows the group combined administration of the VCR (100 μg/kg) and the Compound 1 (100 mg/kg) and, similarly after this, "VCR + Compound 43" shows the group combined administration of the VCR (100 μg/kg) and the Compound 43 (100 mg/kg).

Effect of overcoming resistance to anti-cancer drug (T/V) % = [(Rate of prolongation of life using VCR and present compound (T/C))/(Rate of prolongation of life using VCR alone (T/C))] x 100

Table 12

| | Average days of survival | Rate of prolongation of life T/C (%) | Effect of overcoming resistance T/V (%) |
|---|---|---|---|
| Control | 11.8 | 100 | - |
| VCR alone | 11.3 | 96 | 100 |
| VCR + Compound 1 | 12.8 | 108 | 113 |
| VCR + Compound 3 | 15.0 | 127 | 133 |
| VCR + Compound 16 | 16.2 | 137 | 143 |

Table 13

| | Average days of survival | Rate of prolongation of life T/C (%) | Effect of overcoming resistance T/V (%) |
|---|---|---|---|
| Control | 9.5 | 100 | - |
| VCR alone | 9.8 | 103 | 100 |

Table 13 (continued)

| | Average days of survival | Rate of prolongation of life T/C (%) | Effect of overcoming resistance T/V (%) |
|---|---|---|---|
| VCR + Compound 23 | 13.5 | 142 | 138 |
| VCR + Compound 24 | 13.3 | 140 | 136 |
| VCR + Compound 43 | 13.4 | 141 | 137 |

Table 14

| | Average days of survival | Rate of prolongation of life T/C (%) | Effect of overcoming resistance T/V (%) |
|---|---|---|---|
| Control | 11.3 | 100 | - |
| VCR alone | 12.3 | 109 | 100 |
| VCR + Compound 15 | 15.7 | 139 | 128 |

Test Example 4

Action Easing KCl Contraction in Rat Arteries

An approximately 2 mm long ring specimen was taken from the thoracic aorta of the rat and suspended in a Magnus tube filled with Krebs solution of 37°C aerated with 95% $O_2$-5% $CO_2$. The specimen was stabilized for approximately 60 minutes, then a potassium chloride solution was added to the Magnus tube to give a final concentration of 50mM. The positive control compound or the compound of the present invention was cumulatively added from 1 x $10^{-10}$M to 1 x $10^{-5}$M when the contraction reaction occurring at that time reached equilibrium. The contraction force was recorded by an FD-pickup (Nihon Koden) via a polygraph (Nihon Koden). The results are shown in Table 15 as the 50% inhibitory concentration ($IC_{50}$ value) of hyperpotassium contraction, together with the result of the positive control compound, Nicardipine.

Table 15

| Positive control compound and invention compound | $IC_{50}$ value (x $10^{-6}$ M) |
|---|---|
| Nicardipine | 0.26 |
| Verapamil | 1.43 |
| Compound 2 | >30.0 |
| Compound 3 | >30.0 |
| Compound 4 | 17.4 |
| Compound 10 | 17.1 |
| Compound 11 | >30.0 |
| Compound 14 | 27.1 |
| Compound 15 | >30.0 |

Test Example 5

Acute Toxicity Test

Animals used: ddY male mice (SLC Japan), 4-5 weeks old, three to five mice per group.
Test method: The compound of the present invention was suspended in 0.5% sodium carboxymethylcellulose (CMC-Na) containing 0.1% Tween 80. It was administered to the mice intraperitoneally in amounts from 1000 mg/kg to 250 mg/kg in groups of three to five mice until no more deaths were observed. The survival of the animals was observed up until 7 days after administration.

The test results are shown below:

| | $LD_{50}$ value |
|---|---|
| Compound 1 | >1000 mg/kg |
| Compound 2 | >1000 mg/kg |
| Compound 16 | >1000 mg/kg |
| Compound 23 | >1000 mg/kg |
| Compound 24 | >1000 mg/kg |
| Compound 33 | >1000 mg/kg |

INDUSTRIAL APPLICABILITY

The 1,4-dihydropyridine derivative according to the present invention reverses the action of anti-cancer drugs when used in combination. The effects are particularly remarkable against cells acquiring resistance to anti-cancer drugs. For example, as clear from the above Table 11, human rhinopharynx cancer derived KB cell multi-drug resistant clone VJ-300 cells, compared with cells not acquiring resistance, required use of 483 to 952 times the concentration of the anti-cancer drug to obtain the same effect (50% cell survival rate), while with the combined use of the Compound 2 of the present invention (1 μg/ml), the same effect was obtained with 9.6 times the concentration.
Further, the compound according to the present invention is low in toxicity and exhibits its effects in tests both in vitro and in vivo, and therefore, is useful as a drug for overcoming resistance to an anti-cancer drug or a drugs for increasing the effect of an anti-cancer drug.

**Claims**

**1.** A 1,4-dihydropyridine derivative having the formula (I):

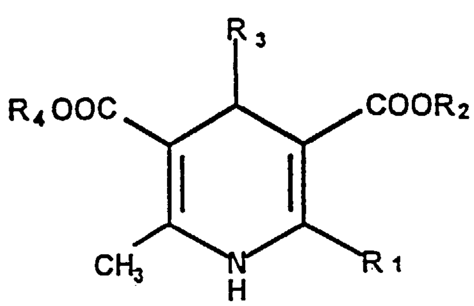

(I)

wherein $R_1$ represents a substituted or unsubstituted phenyl or heterocyclic group, $R_2$ represents a $C_1$ to $C_5$

lower alkyl group, $R_3$ represents a substituted or unsubstituted $C_2$ to $C_8$ alkyl, alkenyl or alkynyl group, or a substituted or unsubstituted cycloalkyl group, $R_4$ represents -A-$R_5$, wherein A represents a $C_2$ to $C_8$ alkylene group or a substituted or unsubstituted $C_2$ to $C_8$ alkenylene group, and $R_5$ represents a substituted or unsubstituted pyridyl, pyridylcarbonyl or piperadinyl group.

**2.** A 1,4-dihydropyridine derivative as claimed in claim 1, wherein $R_1$ is a substituted or unsubstituted phenyl or pyridyl group.

**3.** A 1,4-dihydropyridine derivative as claimed in claim 1, wherein $R_1$ is a phenyl group substituted with one heterocyclic group selected from the group consisting of imidazopyridine, piperadine, imidazole, morpholine, indole, benzimidazole, and 1H-benzotriazole.

**4.** A 1,4-dihydropyridine derivative as claimed in claim 1, wherein $R_1$ is a phenyl group, 3-pyridyl group, 4-(2-methylimidazo[4,5-b]pyridin-1-yl)phenyl group, 4-(2-methylimidazo[4,5-c]pyridin-1-yl)phenyl group, 4-(4-methylpiperadin-1-yl)phenyl group, 4-methylthiophenyl group, 4-(1H-benzotriazol-1-yl)phenyl group, 4-(benzimidazoyl-1-yl)phenyl group, 4-(1-imidazolyl)phenyl group, 3-(1-imidazolyl)phenyl group, 4-morpholinophenyl group or 4-(1-indolyl)phenyl group.

**5.** A 1,4-dihydropyridine derivative as claimed in any one of claims 1 to 3, wherein $R_2$ is a methyl group, ethyl group, or propyl group.

**6.** A 1,4-dihydropyridine derivative as claimed in any one of claims 1 to 4, wherein $R_3$ is a $C_3$ to $C_6$ alkyl or alkenyl group; a $C_1$ to $C_3$ lower alkyl group, a $C_2$ to $C_3$ lower alkenyl group, or a $C_2$ to $C_3$ lower alkynyl group substituted with a phenyl, thienyl, furyl, cyclohexyl or cyclohexylidene group; or a cycloalkyl group.

**7.** A 1,4-dihydropyridine derivative as claimed in any one of claims 1 to 6, wherein $R_4$ represents -A-$R_5$, wherein A is a trimethylene, propenylene or pentamethylene group, and $R_5$ is a 3-pyridyl, 3-pyridylcarbonyl or 4-benzhydrylpiperadin-1-yl group.

**8.** A pharmaceutical composition comprising a 1,4-dihydropyridine derivative according to any one of claims 1 to 7 or its pharmacologically acceptable salt or hydrate and a carrier.

**9.** A drug for overcoming resistance to an anti-cancer drug comprising a 1,4-dihydropyridine derivative according to any one of claims 1 to 7 or its pharmacologically acceptable salt or hydrate as an effective ingredient.

**10.** A drug for increasing the effect of an anti-cancer drug comprising a 1,4-dihydropyridine derivative according to any one of claims 1 to 7 or its pharmacologically acceptable salt or hydrate as an effective ingredient.

## INTERNATIONAL SEARCH REPORT

International application No.
PCT/JP97/04287

**A. CLASSIFICATION OF SUBJECT MATTER**

Int. Cl[6] C07D401/12, 14, 211/90, 471/04, A61K31/445, 495, 535

According to International Patent Classification (IPC) or to both national classification and IPC

**B. FIELDS SEARCHED**

Minimum documentation searched (classification system followed by classification symbols)

Int. Cl[6] C07D401/12, 14, 211/90, 471/04, A61K31/445, 495, 535

Documentation searched other than minimum documentation to the extent that such documents are included in the fields searched

Electronic data base consulted during the international search (name of data base and, where practicable, search terms used)

CA, REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| A | JP, 58-152816, A (Japanese Foundation For Cancer Research), Septemper 10, 1983 (10. 09. 83), Claims (Family: none) | 1 - 10 |
| A | JP, 1-156959, A (Banyu Pharmaceutical Co., Ltd.), June 20, 1989 (20. 06. 89), Claims (Family: none) | 1 - 10 |
| A | JP, 1-316357, A (Ajinomoto Co., Inc.), December 21, 1989 (21. 12. 89), Claims & EP, 330470, A & US, 5216172, A | 1 - 10 |
| P,A | JP, 8-310950, A (Nikken Chemicals Co., Ltd.), November 26, 1996 (26. 11. 96), Claims & WO, 96/4268, A1 & EP, 801065, A | 1 - 10 |

☐ Further documents are listed in the continuation of Box C. ☐ See patent family annex.

* Special categories of cited documents:
- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| February 3, 1998 (03. 02. 98) | February 10, 1998 (10. 02. 98) |
| Name and mailing address of the ISA/ Japanese Patent Office | Authorized officer |
| Facsimile No. | Telephone No. |

Form PCT/ISA/210 (second sheet) (July 1992)